(19) Europäisches Patentamt
European Patent Office
Office européen des brevets

(11) Veröffentlichungsnummer: **0 433 853 B1**

# (12) EUROPÄISCHE PATENTSCHRIFT

(45) Veröffentlichungstag der Patentschrift: **16.08.95**

(51) Int. Cl.[6]: **C12Q 1/00**, C12Q 1/34, C12Q 1/40, C12Q 1/42, C12Q 1/44

(21) Anmeldenummer: **90123834.5**

(22) Anmeldetag: **11.12.90**

(54) **Verwendung von 1-Arylsemicarbaziden zur Stabilisierung von Enzymsubstraten, entsprechende Verfahren und diagnositsches Mittel enthaltend einen solchen Stabilisator.**

(30) Priorität: **21.12.89 DE 3942356**

(43) Veröffentlichungstag der Anmeldung:
**26.06.91 Patentblatt 91/26**

(45) Bekanntmachung des Hinweises auf die Patenterteilung:
**16.08.95 Patentblatt 95/33**

(84) Benannte Vertragsstaaten:
**AT BE CH DE DK ES FR GB GR IT LI LU NL SE**

(56) Entgegenhaltungen:
**EP-A- 0 274 700**
**DE-A- 2 716 060**

(73) Patentinhaber: **BOEHRINGER MANNHEIM GMBH**

**D-68298 Mannheim (DE)**

(72) Erfinder: **Mangold, Dieter, Dipl.Chem. Dr.**
**Hüttenmüllerstrasse 33**
**W-6701 Maxdorf (DE)**

EP 0 433 853 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung eines 1-Arylsemicarbazides zur Stabilisierung eines Enzymsubstrates, aus dem durch enzymatische Hydrolyse ein Leukofarbstoff gebildet werden kann, der seinerseits durch ein Oxidationsmittel zu einem Farbstoff umgesetzt werden kann.

Weiter betrifft die Erfindung ein Verfahren zur Stabilisierung eines wie vorstehend charakterisierten Enzymsubstrats.

Schließlich betrifft die Erfindung ein diagnostisches Mittel zur kolorimetrischen Bestimmung eines Enzyms enthaltend ein Enzymsubstrat aus dem durch enzymatische Hydrolyse ein Leukofarbstoff gebildet werden kann, der seinerseits durch ein Oxidationsmittel zu einem Farbstoff umgesetzt werden kann.

Unter Hydrolasen werden Enzyme verstanden, welche Bindungen unter Wasserverbrauch, hydrolytisch, spalten. In der klinischen Chemie und in der Diagnostik hat in den letzten Jahren vor allem die Bestimmung der Aktivität solcher Hydrolasen an Bedeutung gewonnen, welche Ester- und Etherbindungen spalten. Zu nennen sind hier beispielhaft: Esterasen, wie z. B. die in Leukocyten vorkommenden Carboxylester spaltenden Enzyme oder Phosphatasen, wie z. B. alkalische oder saure Phosphatasen, die Phosphorsäureester hydrolisieren. Für die Diagnostik von Erkrankungen der Niere und des Urogenitaltraktes hat es sich als nützlich erwiesen, Leukozyten anhand der ihnen innewohnenden esterolytischen Aktivität in Urin nachzuweisen. Die Aktivitätsbestimmung der sauren Phosphatase ist ein wertvolles Mittel zur Frühdiagnose von Prostata-Karzinomen. Alkalische Phosphatase kann als Markerenzym für Enzymimmunoassays eingesetzt werden.

Glykosidasen, wie z. B. Galactosidasen, Glucosidasen, Mannosidasen, Amylase oder N-Acetyl-β-D-glucosaminidase spalten glykosidische Bindungen. Sie erfüllen im menschlichen und tierischen Organismus eine vielfache physiologische Funktion. So spielt beispielsweise die β-D-Galactosidase eine gewichtige Rolle im Kohlenhydratstoffwechsel, da durch sie die Hydrolyse der Lactose erfolgt. Darüber hinaus stellt die β-D-Galactosidase das Schlüsselenzym beim Abbau von Glycolipiden, Mucopolysacchariden und Glycoproteinen dar. Als weitere physiologisch bedeutsame Glycosidasen sind zu nennen: die α-D-Galactosidase, die α-D- und β-D-Glucosidase sowie die α-D-Mannosidase.

Über ihren physiologischen Stellenwert hinaus haben die Glycosidasen in den letzten Jahren im diagnostischen sowie im biotechnologischen Bereich an Bedeutung gewonnen. So werden beispielsweise diese Enzyme in zunehmendem Maße als Indikator-Enzym für Enzymimmunoassays eingesetzt. Besonders bevorzugt wird in diesem Zusammenhang die β-D-Galactosidase.

Das Vorhandensein des Enzyms N-Acetyl-β-D-glucosaminidase (β-NAGase) in Körperflüssigkeiten ist ein wertvoller Indikator für Krankheiten oder Fehlfunktionen im Organismus. Im Harn sind z. B. erhöhte Werte bei Nierenübertragungen ein Zeichen für die Abstoßung der Spenderniere. Erhöhte Werte treten ebenso auf bei einer Vielzahl von Krankheitsbildern und von toxischen Schädigungen der Niere. Im Speichel von Frauen ist die NAGase-Aktivität ein Indikator für Fruchtbarkeit und Schwangerschaft.

Zum Nachweis von Hydrolasen können diese mit einem geeigneten Substrat versetzt werden, das enzymatisch gespalten wird und hierbei ein nur schwach gefärbtes oder vorzugsweise ein farbloses Spaltprodukt freisetzt, das durch Oxidation in einen Farbstoff überführt werden kann. Das aus dem Substrat freigesetzte nur schwach gefärbte oder vorzugsweise farblose Spaltprodukt wird als Leukofarbstoff bezeichnet. Entsprechende Substrate und Hydrolasebestimmungsverfahren sind aus dem Stand der Technik bekannt, beispielsweise aus EP-A-0 274 700.

Allgemein können vorstehende Bestimmungsverfahren durch folgendes Reaktionsschema dargestellt werden:

$$\text{G-O-L} \xrightarrow[\text{H}_2\text{O}]{\text{Hydrolase}} \text{G-OH} + \text{HO-L}$$

$$\text{HO-L} \xrightarrow{\text{Oxidationsmittel}} \text{F}$$

G-O-L bezeichnet das Hydrolase-Substrat, aus dem unter Einwirkung eines entsprechenden Hydrolase-Enzyms in Anwesenheit von Wasser Spaltprodukte entstehen. G-OH kann eine entsprechende Säure oder

ein Alkohol, wie z. B. ein Zucker sein. HO-L ist ein Leukofarbstoff, der zu einem Farbstoff, F, oxidiert werden kann.

In der Regel sind Hydrolase-Substrate der vorgenannten Art schwieriger zu oxidieren, als die aus ihnen durch Hydrolyse freisetzbaren Leukofarbstoffe. Dies ist insbesondere auch deshalb wichtig, weil diese Hydrolase-Substrate meist zusammen mit Oxidationsmitteln eingesetzt und auch oft in Kontakt mit diesen aufbewahrt und gelagert werden.

Substrate, wie sie vorstehend dargestellt sind, werden vorteilhaft dort eingesetzt, wo das kolorimetrische Bestimmungsverfahren von nicht gefärbten Substraten ausgehen und zu stark gefärbten Endprodukten einer enzymatischen Spaltung führen soll. Dies ist z. B. dann der Fall, wenn das Substrat in hohen Konzentrationen, beispielsweise auf Teststreifen, eingesetzt wird und/oder wenn eine möglichst hohe Empfindlichkeit des Bestimmungsverfahrens erreicht werden soll. Werden Substrate, die selbst schon gefärbt sind, noch dazu in hohen Konzentrationen eingesetzt, dann kann diese Eigenfärbung eine bei geringer Enzymkonzentration nur schwache Verfärbung durch die Bestimmungsreaktion verdecken und damit zu einem unempfindlichen Test führen. Die Vorteile von ungefärbten Hydrolase-Substraten bleiben verständlicherweise deshalb nur solange erhalten, wie die Substrate auch bei längerer Lagerung ungefärbt bleiben und sich unter den Lagerungsbedingungen nicht verfärben. Es muß deshalb das Bestreben sein, solche Hydrolase-Substrate in einer möglichst lagerstabilen Form aufzubewahren, so daß auch längere Aufbewahrungen und/oder höhere Temperaturen nicht zu Verfärbungen führen.

Benötigt werden folglich Stabilisatoren für Hydrolase-Substrate der beschriebenen Art, die a) selbst gut lagerfähig sind und sich lange Zeit, u. U. auch bei erhöhter Temperatur ohne Verfärbung aufbewahren lassen, die b) durch gleichzeitig anwesende Oxidationsmittel, wie beispielsweise Jodat nicht oxidiert werden und die c) die Oxidation von aus dem Hydrolase-Substrat freigesetztem Leukofarbstoff nicht behindern.

Aus DE-A-27 16 060 sind 1-Arylsemicarbazide bekannt, die Oxidationsindikatoren gegen Umwelteinflüsse wie Luft und Licht stabilisieren. Diese Oxidationsindikatoren dienen zum kolorimetrischen Nachweis von Wasserstoffperoxid. Sie werden deshalb verständlicherweise nicht in Anwesenheit starker Oxidationsmittel aufbewahrt. Die Stabilisierung der Oxidationsindikatoren verhindert nicht die bestimmungsgemäße Reaktion der Indikatoren mit dem nachzuweisenden Oxidationsmittel.

Gegenüber diesem Stand der Technik war es überraschend festzustellen, daß 1-Arylsemicarbazide auch in der Lage sind, Hydrolase-Substrate der eingangs beschriebenen Art zu stabilisieren, die selbst keine Oxidationsindikatoren darstellen, da sie ja gerade nicht in Anwesenheit starker Oxidationsmittel reagieren sollen, auf der anderen Seite aber nach Hydrolyse durch das zu bestimmende Enzym durch Oxidation der entsprechenden Leukofarbstoffe die Anwesenheit dieses Enzyms durch Farbbildung ungehindert anzeigen müssen.

In DE-A-27 16 060 wird die Stabilisierung von Oxidationsindikatoren mittels Arylsemicarbaziden beschrieben. Die darin beschriebenen Oxidationsindikatoren sind jedoch keine Hydrolasesubstrate.

In EP-A-0 274 700 sind Hydrolasesubstrate auf der Basis von Dihydroresorufinen beschrieben. Eine Stabilisierung dieser Verbindungen ist nicht beschrieben.

Die erfindungsgemäße Stabilisierung von Hydrolase-Substraten, aus denen durch enzymatische Hydrolyse ein Leukofarbstoff gebildet werden kann, der seinerseits durch ein Oxidationsmittel zu einem Farbstoff umgesetzt werden kann, ist in den Patentansprüchen charakterisiert.

Gegenstand der Erfindung ist die Verwendung eines 1-Arylsemicarbazides der allgemeinen Formel I

$$Ar\text{-}NH\text{-}NH\text{-}CONH_2 \qquad (I)$$

in der

Ar einen gegebenenfalls durch Alkyl, Alkoxy oder Halogen substituierten Arylrest bedeutet, zur Stabilisierung eines Enzymsubstrats, aus dem durch enzymatische Hydrolyse ein Leukofarbstoff gebildet werden kann, der seinerseits durch ein Oxidationsmittel zu einem Farbstoff umgesetzt werden kann.

Ein weiterer Gegenstand der Erfindung ist ein Verfahren zur Stabilisierung eines Enzymsubstrats, aus dem durch enzymatische Hydrolyse ein Leukofarbstoff gebildet werden kann, der seinerseits durch ein Oxidationsmittel zu einem Farbstoff umgesetzt werden kann, dadurch gekennzeichnet, daß das Substrat in Kontakt mit einem 1-Arylsemicarbazid der allgemeinen Formel I

$$Ar\text{-}NH\text{-}NH\text{-}CONH_2 \qquad (I)$$

in der

Ar einen gegebenenfalls durch Alkyl, Alkoxy oder Halogen substituierten Arylrest bedeutet, gebracht

wird.

Schließlich ist Gegenstand der Erfindung ein diagnostisches Mittel zur kolorimetrischen Bestimmung eines Enzyms enthaltend ein Enzymsubstrat aus dem durch enzymatische Hydrolyse ein Leukofarbstoff gebildet werden kann, der seinerseits durch ein Oxidationsmittel zu einem Farbstoff umgesetzt werden kann, dadurch gekennzeichnet, daß es zur Stabilisierung des Enzymsubstrats ein 1-Arylsemicarbazid der allgemeinen Formel I

$$Ar\text{-}NH\text{-}NH\text{-}CONH_2 \qquad (I)$$

in der

Ar einen gegebenenfalls durch Alkyl, Alkoxy oder Halogen substituierten Arylrest bedeutet,

enthält.

"Aryl" in der Bedeutung der 1-Arylsemicarbazide der allgemeinen Formel I bezeichnet einen Kohlenstoffaromatenrest, vorzugsweise einen solchen mit 6 - 10 Ringatomen, insbesondere die Phenyl- und Naphthylgruppe.

"Alkyl" bedeutet einen geradkettigen oder verzweigten Alkylrest mit 1 - 6, vorzugsweise 1 - 4 C-Atomen. Beispiele sind die Methyl-, Ethyl-, Propyl-, iso-Butyl- oder tert.-Butylgruppe. Bevorzugt sind der Methyl- und Ethylrest.

"Alkoxy" steht für einen geradkettigen oder verzweigten Alkoxyrest mit 1 - 6, vorzugsweise 1 - 4 C-Atomen. Beispiele sind die Methoxy-, Ethoxy-, Propyloxy-, iso-Butyloxy- oder tert.-Butyloxygruppe. Bevorzugt sind der Methoxy- und Ethoxyrest.

"Halogen" bedeutet Fluor, Chlor, Brom oder Jod, vorzugsweise Chlor.

Besonders vorteilhaft haben sich für die erfindungsgemäße Verwendung o-Chlor-, m-Chlor-, p-Chlor-, o-Methyl-, m-Methyl, p-Methyl-, o-Methoxy-, m-Methoxy-, p-Methoxy- und unsubstituiertes Phenylsemicarbazid und Naphthylsemicarbazid erwiesen. Ganz besonders geeignet sind Methylphenylsemicarbazide und Phenylsemicarbazid. Besonders bevorzugt ist p-Methylphenylsemicarbazid.

Die erfindungsgemäß eingesetzten 1-Arylsemicarbazide der allgemeinen Formel I erhöhen nicht nur die Lagerfähigkeit von Hydrolase-Substraten der eingangs genannten Art in Abwesenheit, sondern vor allem auch in Anwesenheit starker Oxidationsmittel, wie Kaliumhexacyanoferrat (III), Perborat, Peroxidase/Wasserstoffperoxid oder vorzugsweise einem Jodat, wie Kaliumjodat.

Der Effekt der Stabilisierung bezieht sich auf in Lösung, vor allem in wässriger Lösung befindliche Substanzen, insbesondere aber auf feste, ungelöste Hydrolase-Substrate, gegebenenfalls im Gemisch mit einem Oxidationsmittel und festem ungelöstem 1-Arylsemicarbazid, seien es beispielsweise Kristalle, Pulver, Lyophilisate oder in Tablettenform gepreßte feste Substanzen. Eine Stabilisierung in Form der Verhinderung einer Verfärbung von Hydrolase-Substraten ist vorzugsweise bei Testträgern möglich, bei denen Hydrolase-Substrat und Oxidationsmittel trägergebunden entweder zusammen auf oder in einem saugfähigen oder quellbaren Trägermaterial wie Papier oder einem Polymerfilm vorliegen oder bei denen Hydrolase-Substrat und Oxidationsmittel in oder auf getrennte Trägermaterialien vorliegen, die jedoch ihrerseits miteinander in direkter Berührung und in Kontakt zueinander stehen.

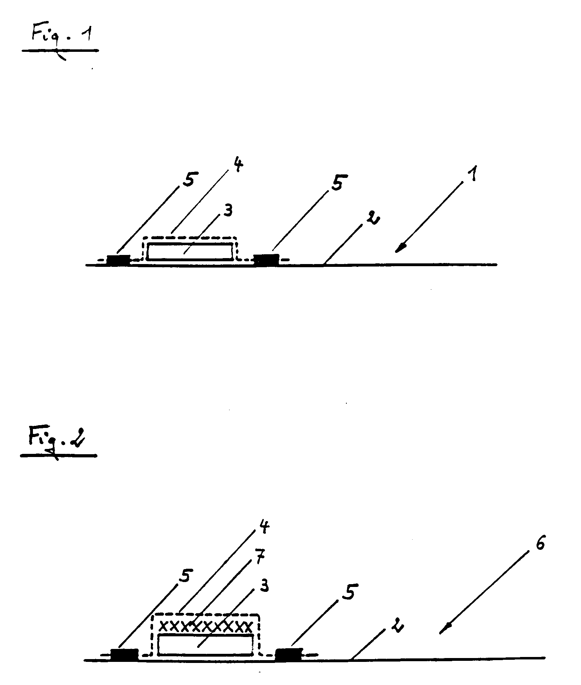

Beispiele für Testträger zur Bestimmung von Hydrolasen sind beispielsweise in Figuren 1 und 2 dargestellt.

Fig. 1 zeigt die Seitenansicht eines Testträgers (1) zur kolorimetrischen Bestimmung eines Hydrolase-Enzyms. Auf einer Trägerfolie (2), vorzugsweise aus steifem Kunststoff, ist ein saugfähiger Reagenzträger (3) mittels eines Abdecknetzes (4), das mit Schmelzkleber (5) auf der Trägerfolie (2) befestigt ist, fixiert. Der saugfähige Reagenzträger (3) kann beispielsweise aus Papier bestehen, das mit den für die Bestimmung notwendigen Reagenzien, insbesondere Hydrolase-Substrat, Oxidationsmittel und Stabilisator imprägniert ist. Die Reagenzien können auf einmal, d. h. in einem Schritt, aus einer Lösung auf den Reagenzträger (3) aufgebracht worden sein, oder sie können in mehreren Schritten durch Tränkung aus getrennten Lösungen aufimprägniert worden sein. Vorzugsweise wird der Reagenzträger (3) erst mit einer Lösung des Oxidationsmittels, das gegebenenfalls in einer den für die Bestimmungsreaktion notwendigen pH-Wert einstellenden Pufferlösung gelöst ist, imprägniert, dann getrocknet und anschließend mit einer Lösung, die das Hydrolase-Substrat und den erfindungsgemäßen Stabilisator enthält, ein zweites Mal imprägniert.

Fig. 2 zeigt ebenfalls die Seitenansicht eines Testträgers (6) zur kolorimetrischen Bestimmung eines Hydrolase-Enzyms. Er ist ähnlich wie der Testträger gemäß Fig. 1 aufgebaut. Er enthält jedoch unter dem Abdecknetz (4), das mit Schmelzkleber (5) auf einer Trägerfolie (2) befestigt ist, außer einem saugfähigen Reagenzträger (3) ein Netz (7). Dieses Netz, das vorzugsweise aus Polymermaterial besteht, ist mit dem Oxidationsmittel imprägniert. Der Reagenzträger (3) enthält Hydrolase-Substrat, Stabilisator und gegebenenfalls Puffersubstanz zur Einstellung eines zur Durchführung der Bestimmungsreaktion notwendigen pH-Wertes.

Wird auf das Abdecknetz (4) der Testträger gemäß Fig. 1 oder 2 eine Hydrolase-haltige Lösung aufgegeben oder die Testträger in eine solche Lösung eingetaucht, dringt die Probe schnell durch das Abdecknetz (4) und unter Lösung der imprägnierten Reagenzien in den saugfähigen Reagenzträger (3) ein. Dort findet bei Anwesenheit von Hydrolase die eingangs beschriebene Reaktionsfolge statt, an deren Ende ein Farbstoff gebildet wird, der auf den Testträgern visuell festgestellt oder remissionsphotometrisch vermessen werden kann und so Aufschluß über die Konzentration des zu bestimmenden Enzyms in der zu untersuchenden Probe gibt.

Es hat sich gezeigt, daß bei erfindungsgemäßer Verwendung der 1-Arylsemicarbazide der allgemeinen Formel I eine bemerkenswerte Stabilisierung von Hydrolase-Substraten der eingangs beschriebenen Art stattfindet. Diese Stabilisierung läßt sich vor allem an der wesentlich verringerten oder völlig fehlenden Verfärbung solcher Hydrolase-Substrate feststellen, wenn sie allein oder zusammen mit Oxidationsmitteln, wie beispielsweise Jodat, insbesondere bei erhöhter Temperatur und Lichteinwirkung aufbewahrt werden. Vorzugsweise werden zur Erzielung einer Stabilisierung Hydrolase-Substrat und 1-Arylsemicarbazid so gemischt, daß nach Kontaktierung mit der zu untersuchenden Flüssigkeit die Konzentration der 1-Arylsemicarbazide der allgemeinen Formel I in der Probenflüssigkeit 0,25 - 50 mMol/l, besonders bevorzugt 0,5 -20 mMol/l beträgt, während die Konzentration des Enzymsubstrats unter den gleichen Bedingungen 2,5 - 50 mMol/l, besonders bevorzugt 5 - 30 mMol/l ist. Wenn die Reagenzien auf Testträgern wie beispielsweise solchen gemäß Fig. 1 und 2 eingesetzt werden, entsprechen die vorgenannten Konzentrationen auch den Konzentrationen der zur Imprägnierung der verwendeten Trägermaterialien verwendeten Lösungen.

Der stabilisierende Effekt der 1-Arylsemicarbazide der allgemeinen Formel I auf Hydrolase-Substrate der eingangs beschriebenen Art zeigt sich nicht nur im neutralen und alkalischen Milieu, sondern vor allem auch im sauren pH-Bereich ab etwa pH 3,5.

Insbesondere bei Testträgern gemäß Fig. 2 hat es sich gezeigt, daß die Konzentration an Arylsemicarbazid verringert werden kann, ohne die Stabilisierung des Hydrolase-Substrates zu vermindern, wenn das Reagenzpapier (3) neben dem Hydrolase-Substrat und 1-Arylsemicarbazid Ascorbinsäure oder Thiosulfat enthält. Es ist hierbei jedoch darauf zu achten, daß die Menge an Oxidationsmittel in Netz (7) so groß ist, daß bei Probenaufgabe und folgender Reagenzkomponentenvermischung die Ascorbinsäure oder das Thiosulfat sehr schnell oxidiert wird und dann noch ausreichend Oxidationsmittel zur Oxidation enzymatisch gebildeten Leukofarbstoffs zur Verfügung steht. Durch eine solche Ascorbinsäure- oder Thiosulfatzugabe kann nicht nur die 1-Arylsemicarbazid-Konzentration gesenkt werden. Es ist außerdem überraschenderweise eine schnellere Farbentwicklung bei der Hydrolase-Bestimmung zu beobachten.

Als effektiv hat sich die Verwendung von 1-Arylsemicarbaziden der allgemeinen Formel I für die Stabilisierung vor allem solcher Hydrolase-Substrate erwiesen, die durch Hydrolyse 3-Hydroxy-indol liefern oder ein im Indolkern substituiertes 3-Hydroxy-indol, beispielsweise 5-Brom-4-chlor-3-hydroxyindol.

Die Verwendung von 1-Arylsemicarbaziden der allgemeinen Formel I hat sich insbesondere für die Stabilisierung von Hydrolase-Substraten der allgemeinen Formel II

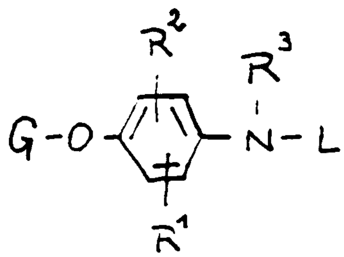

(II)

bewährt, in der

G einen organischen oder anorganischen Säurerest oder einen Glycosidrest darstellt,

$R^1$ und $R^2$, die gleich oder verschieden sind, Wasserstoff, Halogen, $SO_3H$, $PO_3H_2$ oder ein Salz dieser Säuregruppe, eine Hydroxy-, Nitro-, Carboxy-, Carboxamido- oder Cyanogruppe oder eine gegebenenfalls durch einen oder mehrere Hydroxy-, Carboxy-, Halogen-, Cyano-, $SO_3H$- oder $PO_3H_2$-Reste oder ein Salz eines dieser Säurereste substituierte Alkyl-, Alkenyl-, Alkoxy-, Alkylsulfinyl-, Alkylsulfonyl-, Alkoxycarbonyl-, Alkylcarbonyl-, Aryl-oder Aralkylgruppe bedeuten oder
wenn sich beide Reste an benachbarten Kohlenstoffatomen befinden gemeinsam eine 1,4-Butadiendiylgruppe darstellen, die gegebenenfalls ein-oder mehrmals durch $SO_3H$,

$PO_3H_2$ oder ein Salz dieser Säuregruppen, eine Alkyl- oder/und eine Carboxygruppe substituiert ist,

$R^3$ Wasserstoff, CO-COOH, $SO_3H$, $PO_3H_2$ oder ein Salz dieser Säuregruppen, eine gegebenenfalls ein- oder mehrfach durch Halogen, $CO_2H$, $SO_3H$ oder/und $PO_3H_2$ oder ein Salz dieser Säuregruppen substituierte Alkylcarbonylgruppe oder eine gegebenenfalls ein- oder mehrfach durch $SO_3H$, $PO_3H_2$ oder ein Salz dieser Säurereste substituierte Arylcarbonylgruppe ist und

L einen Rest der allgemeinen Formel III

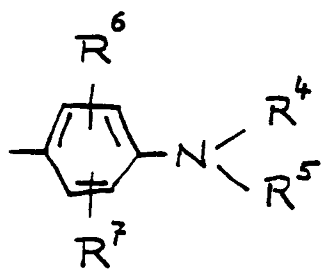

wobei

$R^4$ und $R^5$, die gleich oder verschieden sein können, Alkyl bedeuten oder zusammen eine gesättigte Kohlenwasserstoffkette mit 3 - 6 Gliedern darstellen, die durch Sauerstoff, Schwefel oder Stickstoff unterbrochen sein kann, wobei Alkyl bzw. die Kohlenwasserstoffkette gegebenenfalls ein- oder mehrfach durch eine Hydroxy-, Carboxy-, Alkoxycarbonyl-, Alkoxy-, $SO_3H$- oder $PO_3H_2$ -Gruppe, ein Salz eines dieser Säurereste oder Halogen substituiert ist und

$R^6$ und $R^7$, die gleich oder verschieden sein können, Wasserstoff, Halogen, eine Hydroxy- oder Carboxamidogruppe oder eine gegebenenfalls durch einen oder mehrere Hydroxy-, Carboxy-, Halogen-, $SO_3H$- oder $PO_3H_2$ -Reste oder ein Salz eines dieser Säurereste substituierte Alkyl-, Alkoxy-, Alkylcarbonyl-, Alkoxycarbonyl-, Aryl- oder Aralkylgruppe bedeutet oder

L einen Pyrazoloheterocyclus-Rest der allgemeinen Formel IV

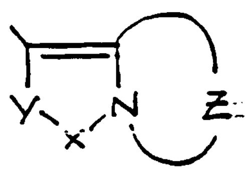

darstellt,
in der
X-Y $NR^8$-CO oder $N=CR^9$ bedeutet,
wobei

$R^8$ Wasserstoff oder Alkyl und

$R^9$ Alkyl, Alkenyl, Alkoxy, Alkylthio, Aryl, Aralkyl, gegebenenfalls jeweils substituiert durch Hydroxy, Dialkylphosphinyl, Carboxy, $SO_3H$, $PO_3H_2$, ein Salz eines dieser Säurereste oder/und Alkoxycarbonyl;
Amino, das gegebenenfalls durch ein oder zwei gegebenenfalls einen oder mehrere Hydroxy-, Carboxy- oder/und Alkoxycarbonylreste tragenden Alkylrest substituiert ist wobei, wenn Amino durch 2 Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann, oder Amino gegebenenfalls durch ein oder zwei Acylgruppen, Alkoxy- oder/und Aralkoxycarbonylgruppen, $H_2N$-CO, Alkyl-, Aralkyl- oder/und Arylcarbamoylgruppen substituiert ist; oder
Wasserstoff, Carboxy, Alkoxycarbonyl, Carboxamido oder Halogen ist, und

Z $NR^{10}$ -N=N bedeutet

wobei $R^{10}$ Wasserstoff, Alkyl oder Aralkyl ist oder

Z eine ungesättigte Kette mit 3 bis 5 Gliedern aus Stickstoffatomen oder aus Kohlenstoffatomen und gegebenenfalls einem oder mehreren Stickstoff- oder Schwefelatomen darstellt, wobei Kohlenstoffatome gegebenenfalls durch Alkyl, Alkoxy, Hydroxyalkyl, Alkylthio, Hydroxy, Aralkyl, Aryl, Carboxy, Carboxamido, Alkoxycarbonyl, Cyano, Amino, das gegebenenfalls durch ein oder zwei gegebenenfalls einen oder mehrere Hydroxy-, Carboxy- oder/und Alkoxycarbonylreste tragenden Alkylrest substituiert ist, oder/und Halogen sowie Stickstoffatome, die nicht über eine Doppelbindung gebunden sind, gegebenenfalls durch Alkyl oder Aralkyl substituiert sind oder zwei benachbarte Kettensubstituenten gegebenenfalls eine Alkylengruppe bilden, die ihrerseits gegebenenfalls mit Aryl substituiert oder anelliert ist oder ein entsprechender tautomerer Rest.

Unter einem anorganischen Säurerest in der Definition von G sind vor allem der Ortho- oder Pyrophosphorsäure- oder Schwefelsäurerest zu verstehen, die über eine Esterbindung an das Aminophenolgrundgerüst gebunden sind. Bevorzugt sind die Reste $PO_3MM'$ und $SO_3M$, insbesondere $PO_3MM'$, wobei im Falle der freien Säuren M und M' Wasserstoff bedeuten, während wenn die Säuren als Salz vorliegen, M und M' für Alkali-, Erdalkali- oder Ammoniumionen stehen.

Unter Alkaliionen in der Definition von M und M' sind vor allem Lithium-, Natrium- und Kaliumionen zu verstehen. Erdalkaliionen sind vor allem Magnesium-, Calcium- oder Bariumionen.

Ammoniumionen in der Definition von M und M' können das unsubstituierte Ammoniumion $NH_4^+$ oder durch Alkyl- oder Aralkylreste ein- oder mehrfach substituierte Ammoniumionen sein. Hierbei ist unter Alkylrest ein Rest aus 1 - 6 Kohlenstoffatomen zu verstehen. Bevorzugt sind der Methyl- oder Ethylrest. Unter einem Aralkylrest ist ein Rest zu verstehen, bei dem eine wie vorstehend definierte Alkylgruppe durch einen Arylrest substituiert ist, wobei Aryl einen Kohlenstoffaromaten- oder Heteroaromatenrest, vorzugsweise einen solchen mit 6 - 10 Ring-Atomen, insbesondere die Phenyl- und die Naphthylgruppe bedeutet. Bevorzugt als Aralkylrest ist der Benzylrest. Die Substituenten substituierter Ammoniumionen können sowohl gleich als auch verschieden sein. Als Ammoniumionen können auch Kationen quaternierter Stickstoffheterocyclen eingesetzt werden. Beispiele hierfür sind das Piperidiniumkation und das Pyridiniumion.

Unter einem organischen Säurerest in der Definition von G werden vor allem Alkancarbonsäure-, Aminosäure- oder Oligopeptidreste verstanden, welche mit ihrem Carboxylende als Ester an das Aminophenolgrundgerüst der allgemeinen Formel II gebunden vorliegen.

Alkancarbonsäurereste in der Definition von G sind Verbindungen mit 1 - 20 Kohlenstoffatomen. Besonders bevorzugt sind Essigsäure, Propionsäure, Buttersäure, Palmitinsäure und Stearinsäure. Neben gesättigten Säureresten kann G auch ein ungesättigter Säurerest, wie z. B. der Ölsäure-, Linolsäure- oder Linolensäurerest sein.

Als Aminosäurerest kommen vorzugsweise die Reste der natürlichen $\alpha$-Aminosäuren in ihrer L- oder D-Form oder auch in ihrer racemischen Form in Frage. Besonders bevorzugt sind die Reste von Glycin, Alanin, Valin, Leucin, Isoleucin, Phenylalanin und Thyrosin, wobei jeweils die L-Form ganz besonders bevorzugt ist.

Unter einem Oligopeptidrest sind z. B. Di-, Tri-, Tetra- und Pentapeptide, vorzugsweise Di- und Tripeptide zu verstehen, wobei als Aminosäurekomponenten vorzugsweise die oben erwähnten Aminosäuren Verwendung finden.

Die Aminogruppen der esterartig an den Aminophenolrest gebundenen Aminosäure- oder Oligopeptidreste können frei oder geschützt vorliegen. Als Schutzgruppe sind hier alle üblichen Aminoschutzgruppen zu verstehen, insbesondere Acyl-, Oxycarbonyl-, Thiocarbonyl-, Sulfo-, Sulfino, Vinyl-, Cyclohexenyl-, Phosphoryl-oder Carbamoyl- Gruppen. Besonders bevorzugte Aminoschutzgruppen sind der Tosyl-, Benzyloxycarbonyl- und tert.-Butoxycarbonyl-Rest.

Ein Glycosidrest in der Definition von G kann ein Mono- oder Oligosaccharid sein. Der Zuckerrest kann $\alpha$- oder $\beta$-glycosidisch an das Aminophenol-Grundgerüst gebunden sein. Beispiele für bevorzugte Monosaccharide sind Galactose, Glucose oder Mannose. Besonders bevorzugt ist N-Acetyl-glucosamin. Ganz besonders bevorzugt ist $\beta$-glycosidisch gebundenes N-Acetyl-2-D-glucosamin.

Als Zuckerreste sind aber auch Oligosaccharide geeignet. Als Oligosaccharide werden insbesondere solche bezeichnet, die aus 2 bis 10, vorzugsweise aus 2 - 7 Monosaccharideinheiten aufgebaut sind. Besonders bevorzugt sind Heptaosen.

Aus der Gruppe der organischen oder anorganischen Säurereste- und der Glycosidreste in der Bedeutung von G sind die Glycosidreste für Verbindungen der allgemeinen Formel II bevorzugt. Insbesondere der N-Acetylglucosaminrest ist ganz besonders bevorzugt, weil solche N-Acetyl-$\beta$-D-glucosaminidase-Substrate durch die 1-Arylsemicarbazide der allgemeinen Formel I besonders gut stabilisiert werden.

Sofern nichts anderes angegeben ist, haben die folgenden Reste in den hier gebrauchten allgemeinen Formeln folgende Bedeutung: "Alkyl" - auch in Alkylsulfinyl-, Alkylsulfonyl-, Alkylcarbonyl, Alkylthio-,

Alkylcarbamoyl-, Alkylamino- und Aralkylresten - bedeutet einen geradkettigen oder verzweigten Alkylrest mit 1-6, vorzugsweise 1-4 C-Atomen. Beispiele sind die Methyl-, Ethyl-, Propyl-, iso-Butyl- oder tert.-Butylgruppe.

Wenn eine Aminogruppe durch 2 Alkylreste substituiert ist, können diese Reste auch so zu einem Ring geschlossen sein, daß sie insgesamt einen durch ein Stickstoffatom unterbrochenen Ring darstellen. Bevorzugt sind hierbei solche Aminogruppen, die einen insgesamt 5- oder 6-gliedrigen Ring darstellen und der seierseits gegebenenfalls durch Sauerstoff, Schwefel oder ein weiteres Stickstoffatom unterbrochen ist. Besonders bevorzugt ist der Morpholinorest. Ein Hydroxyalkylrest ist ein durch eine Hydroxygruppe substituierter Alkylrest mit 1-6, vorzugsweise 1-4 C-Atomen. Der Hydroxyalkylrest kann einen primären, sekundären oder tertiären Alkoholrest darstellen. Besonders bevorzugt sind der 2- oder 1-Hydroxyethyl- und der Hydroxymethylrest.

"Alkoxy" - auch in Alkoxy- und Aralkoxycarbonylresten - steht für einen geradkettigen oder verzweigten Alkoxyrest mit 1 - 6, vorzugsweise 1 - 4 C-Atomen. Beispiele sind die Methoxy-, Ethoxy-, Propyloxy-, iso-Butyloxy- oder tert.-Butyloxygruppe.

"Aryl" - auch in Arylcarbonyl - und Arylcarbamoylresten - bezeichnet einen Kohlenstoffaromaten- oder Heteroaromatenrest, vorzugsweise einen solchen mit 6 - 10 Ring-Atomen, insbesondere die Phenyl- und die Naphthylgruppe, die zusätzlich noch durch Alkyl, Alkoxy oder/und Halogen substituiert sein können. Besonders bevorzugt ist der Phenylrest.

Ein "Aralkyl"-Rest - auch in einer Aralkylcarbamoylgruppe - bedeutet einen Rest, bei dem eine wie vorstehend definierte Alkylgruppe durch einen wie zuvor charakterisierten Arylrest substituiert ist. Bevorzugt ist die Benzylgruppe.

Ein "Aralkoxy"-Rest, beispielsweise in Aralkoxycarbonylgruppen, bezeichnet einen Rest, bei dem eine wie vorstehend definierte Alkoxygruppe durch einen wie zuvor charakterisierten Arylrest substituiert ist. Bevorzugt ist die Benzyloxygruppe.

"Halogen" steht für die Reste Fluor, Chlor, Brom und Jod. Fluor und Chlor sind bevorzugt.

"Alkenyl" bedeutet einen ungesättigten Kohlenwasserstoffrest mit 2 - 6, vorzugsweise 2 - 4 C-Atomen. Beispiele sind die Vinyl- und die Allylgruppe.

Eine Acylgruppe bezeichnet einen Carbonsäurerest, der Alkyl-, Aralkyl- oder Arylreste enthalten kann. Bevorzugt sind Acetyl-, Phenylacetyl- oder Benzoylreste.

Unter einer Alkylengruppe wird eine geradkettige oder verzweigte, gesättigte oder ungesättigte Kohlenwasserstoffkette aus 3 - 5, vorzugsweise 3 oder 4 C-Atomen mit zwei freien Bindungsstellen verstanden.

Beispiele sind $-CH_2-CH=CH-$,

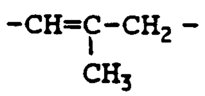 , 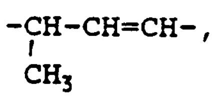 ,

$-(CH_2)_4-$ oder $-CH=CH-CH=CH-$.

Bevorzugt sind der Butadiendiylrest ($-CH=CH-CH=CH-$) und der Tetramethylenrest ($-(CH_2)_4-$). Unter einer Dialkylphosphinylgruppe wird der Rest

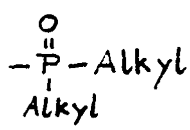

verstanden, wobei Alkyl die zuvor gegebene Bedeutung hat. Bevorzugt ist der Dimethylphosphinylrest.

Als Salze von $SO_3H$, $PO_3H_2$ und Carboxyresten können Alkali- oder Erdalkalisalze oder Ammoniumsalze eingesetzt werden. Unter Alkalisalzen werden Lithium-, Natrium-, Kalium-, Rubidium- und Cäsiumsalze verstanden, wobei Lithium-, Natrium- und Kaliumsalze, vor allem aber Natrium- und Kaliumsalze bevorzugt sind. Erdalkalisalze sind solche des Berylliums, Magnesiums, Calciums, Strontiums oder Bariums. Bevorzugt sind Magnesium- und Calciumsalze, wobei Calciumsalze besonders bevorzugt sind. Als Ammoniumsalze können solche des unsubstituierten Ammoniumions, $NH_4^+$, Verwendung finden. Es ist aber auch möglich, solche Ammoniumsalze einzusetzen, bei denen das Ammoniumion durch 1 - 4 Alkyl-, Aryl- oder Aralkylreste substituiert ist. Für diese Reste gelten die zuvor gegebenen Definitionen, wobei als Alkylrest Methyl, Ethyl und n-Propyl, als Arylrest die Phenylgruppe und als Aralkylrest die Benzylgruppe besonders

bevorzugt sind. Als Ammoniumionen können auch Kationen quaternierter Stickstoffheterocyclen eingesetzt werden. Beispiele hierfür sind das Piperidiniumkation und das Pyridinuimion.

Als Carboxamidorest wird der Rest $CONH_2$ verstanden, aber auch solche Reste, bei denen die Aminogruppe durch ein oder zwei gegebenenfalls einen oder mehrere Hydroxy-, Carboxy- oder/und Alkoxycarbonylreste tragende Alkylreste substituiert ist.

Vorteilhaft sind vor allem solche Hydrolase-Substrate, in denen $R^3$ Wasserstoff oder einen ein- oder mehrfach durch Halogen substituierten Alkylcarbonylrest, insbesondere Trifluoracetyl, bedeutet. Ganz besonders bevorzugt in der Bedeutung von $R^3$ ist Wasserstoff.

Substanzen der allgemeinen Formel II, bei denen L einen Pyrazoloheterocyclus-Rest der allgemeinen Formel IV bedeutet, sind erfindungsgemäß besonders gut stabilisierbar. Hiervon insbesondere solche, in denen Z so angeordnet ist, daß mindestens eine Doppelbindung der ungesättigten Kette in Konjugation zu der Doppelbindung oder zu dem N-Atom der allgemeinen Formel IV steht.

Außerdem sind Substanzen der allgemeinen Formel II besonders bevorzugt, bei denen L einen Pyrazoloheterocyclus-Rest der allgemeinen Formel IV bedeutet, wobei in der ungesättigten Kette Z, falls diese Stickstoffatome enthält, die nicht über eine Doppelbindung gebunden sind, diese mit Alkyl- oder Aralkylresten substituiert sind.

Gegebenenfalls sind für Reste der allgemeinen Formel IV auch tautomere Formen möglich. Diese sollen ebenfalls als von der allgemeinen Formel IV umfaßt gelten.

Erfindungsgemäß bevorzugt stabilisierbar sind solche Substanzen der allgemeinen Formel II, in denen der Rest L einen Rest aus der Gruppe der allgemeinen Formeln V - XVI

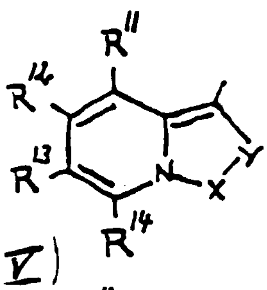

(V)

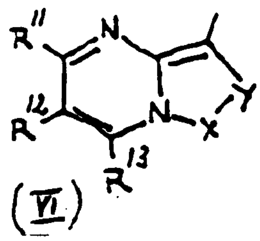

(VI)

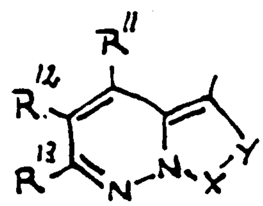

(VII)

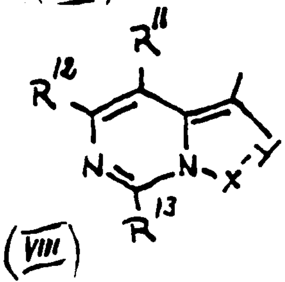

(VIII)

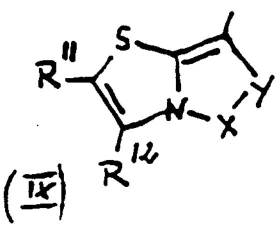

(IX)

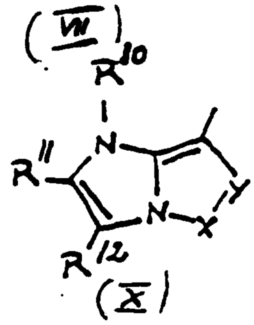

(X)

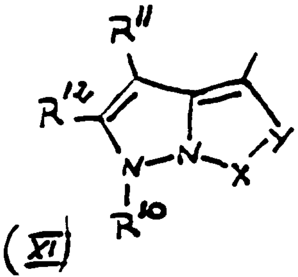

(XI)

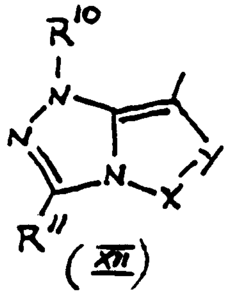

(XII)

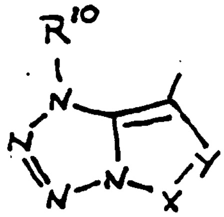

(XIII)

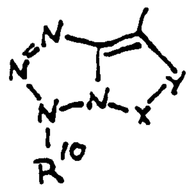

(XIV)

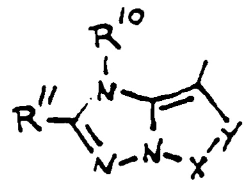

(XV)

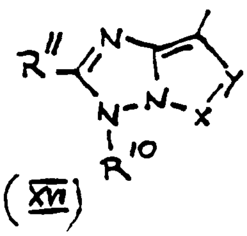

(XVI)

sowie gegebenenfalls entsprechende tautomere Formen bedeutet.

Hierbei haben X-Y und $R^{10}$ die gleiche Bedeutung wie zuvor beschrieben. $R^{11}$, $R^{12}$, $R^{13}$ und $R^{14}$, die gleich oder verschieden sind, stehen für Wasserstoff, Hydroxy, Alkyl, Alkoxy, Alkylthio, Aralkyl, Aryl, Carboxy, Alkoxycarbonyl, Carboxamido, Cyano, Amino, das gegebenenfalls durch ein oder zwei gegebenenfalls einen oder mehrere Hydroxy-, Carboxy- oder/und Alkoxycarbonylreste tragende Alkylreste substituiert ist oder Halogen, wobei zwei benachbarte Reste gegebenenfalls eine Alkylengruppe bilden, die ihrerseits gegebenenfalls mit Aryl substituiert oder anelliert ist. Die Definitionen der Reste entsprechen den zuvor gegebenen.

Erfindungsgemäß besonders bevorzugt stabilisierbare Substanzen sind solche der allgemeinen Formel II in der L für einen Rest aus der Gruppe der allgemeinen Formeln V, VI, VII, IX, X und XII und gegebenenfalls entsprechende tautomere Formen steht. Ganz besonders bevorzugt sind solche Substanzen, in denen X-Y die Bedeutung $N = CR^9$ hat, wobei $R^9$ die Bedeutung haben kann wie für die allgemeine Formel IV angegeben, bevorzugt aber Wasserstoff und Alkoxy darstellt.

Hervorragend stabilisiert im Sinne der vorliegenden Erfindung werden insbesondere die $\beta$-glycosidisch gebundenen N-Acetyl-D-glucosaminide von (4-Hydroxyphenyl)-(2-methylpyrazolo-[1,5-a]-pyridin-3-yl)-amin und (4-Hydroxyphenyl)-(pyrazolo-[1,5-a]-pyridin-3-yl)-amin.

Die Verbindungen der allgemeinen Formel II können hergestellt werden, indem man einen Leukoazomethinfarbstoff der allgemeinen Formel XVII

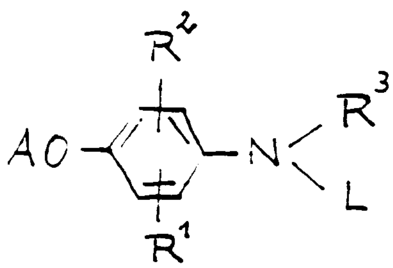

(XVII)

in der $R^1$ -$R^3$ und L die für Formel II gegebene Bedeutung haben und A Wasserstoff, ein gegebenenfalls substituiertes Ammoniumion oder ein Alkalimetall darstellt,
mit einer Verbindung der allgemeinen Formel XVIII

G-D (XVIII)

in der G einen organischen oder anorganischen Säurerest oder einen Glycosidrest in der für Formel II angegebenen Bedeutung darstellt, wobei in dem Glycosidrest sich befindliche funktionelle Gruppen, wie beispielsweise Amino- oder/und Hydroxygruppen gegebenenfalls mit in der Peptid- und Kohlenhydratchemie üblichen Schutzgruppen substituiert sind, und D eine reaktive Gruppe bedeutet,
umsetzt und gegebenenfalls abschließend Schutzgruppen abspaltet.

Unter einem unsubstituierten Ammoniumion wird $NH_4{+}$ verstanden. Gegebenenfalls kann dieses Ion in der Bedeutung von A in der allgemeinen Formel XVII auch durch Alkyl- oder Aralkylreste wie sie zuvor bereits charakterisiert wurden ein- oder mehrfach substituiert sein. Die Substituenten substituierter Ammoniumionen können sowohl gleich als auch verschieden sein. Als Ammoniumionen können auch Kationen quaternierter Stickstoffheterocyclen eingesetzt werden. Beispiele hierfür sind das Piperidiniumkation oder das Pyridiniumion.

Als Alkalimetall in der Bedeutung von A in der allgemeinen Formel XVII kommen insbesondere Lithium, Natrium und Kalium in Frage, wobei Natrium bevorzugt ist.

D bedeutet eine reaktive Gruppe, die in der Lage ist, mit der Phenol- oder Phenolatgruppe OA der allgemeinen Formel XVII eine Reaktion einzugehen. Die Wahl der reaktiven Gruppe ist abhängig von der Natur des Restes G. Handelt es sich bei G um einen Zuckerrest, so ist D vorzugsweise eine gut zu substituierende Gruppe, beispielsweise ein Acetylrest oder ein Halogenatom, welches aus der Gruppe Fluor, Chlor, Brom und Jod ausgewählt werden kann, wobei Chlor, Brom und Jod bevorzugt sind.

Als in der Kohlenhydratchemie übliche Schutzgruppen sind insbesondere der Acetyl-, Benzoyl-, Benzyl- und der Trimethylsilylrest zu nennen.

Wenn G ein Aminosäure- oder Peptidrest bedeutet, der mit seinem Carboxylende mit einem Aminophenol der allgemeinen Formel XVII verestert werden soll, dann kommen als reaktive Gruppe D die in der Peptidchemie üblichen Gruppen in Frage. Als reaktive Derivate werden z. B. die Säurehalogenide, bevorzugt Säurechloride bzw. die bei der Peptidsynthese üblicherweise verwendeten Mischanhydride oder Aktivester eingesetzt. Die gleichen reaktiven Gruppen können auch für die Bindung von Alkancarbonsäuren an das Aminophenol-Gerüst eingesetzt werden.

Für den Fall, daß G einen anorganischen Säurerest bedeutet, werden Verbindungen der allgemeinen Formel XVII vorzugsweise mit den entsprechendeen Säurehalogeniden, insbesondere Säurechloriden umgesetzt.

In jedem Fall ist bei einer Veresterung darauf zu achten, daß falls $R^3$ in Formel XVII Wasserstoff ist, diese Aminogruppe vor Durchführung der Veresterungsreaktion mit einer Schutzgruppe, beispielsweise einer aus der Peptidchemie zu diesem Zweck üblichen Gruppe, substituiert wird und die Schutzgruppe abschließend wieder entfernt wird.

Beispielhaft soll das Verfahren zur Herstellung von Verbindungen der allgemeinen Formel II am Beispiel der besonders bevorzugt stabilisierbaren Verbindungen, in denen G einen N-Acetyl-β-D-glucosaminidrest darstellt, vorgestellt werden. Dieses Verfahren ist entsprechend auch auf die Herstellung anderer Glycosidderivate der allgemeinen Formel II übertragbar.

N-Acetyl-β-D-glucosaminidylderivate der allgemeinen Formel II können hergestellt werden, indem als Verbindung der allgemeinen Formel XVIII
eine Verbindung der allgemeinen Formel XIX

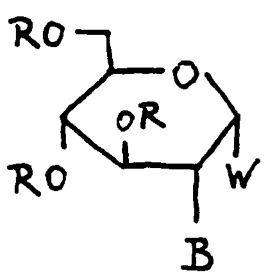

(XIX)

in der
W ein Halogenrest,
R eine in der Kohlenhydratchemime übliche Hydroxyschutzgruppe
und
B ein Azidrest, eine geschützte Aminogruppe oder NH-$COCH_3$ darstellen oder
B und W zusammen die Gruppe

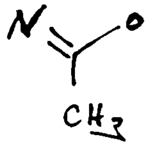

bedeutet,
mit einer Verbindung der allgemeinen Formel XVII

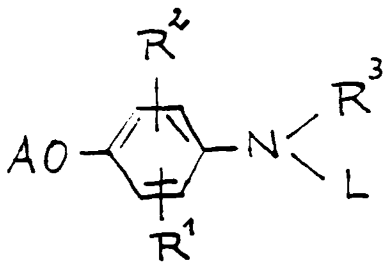

(XVII)

in der

$R^1$-$R^3$ und L die für Formel II angegebene Bedeutung haben und A Wasserstoff, ein gegebenenfalls substituiertes Ammoniumion oder ein Alkalimetall darstellt,

umgesetzt wird,

wenn B eine geschützte Aminogruppe darstellt, die Aminoschutzgruppe entfernt oder

wenn B einen Azidrest darstellt, dieser durch Reduktion in eine $NH_2$-Gruppe überführt und die Aminogruppe durch Acetylierung in den $NHCOCH_3$-Rest umgewandelt wird, und abschließend die Hydroxyschutzgruppen abgespalten werden.

Eine Möglichkeit besteht beispielsweise darin, einen Leukoazomethinfarbstoff der allgemeinen Formel XVII mit der zuvor angegebenen Bedeutung mit einem per-O-substituierten 1-Halogen-N-acetyl-Glucosamin der Formel XIX,

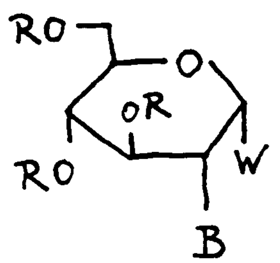

(XIX)

in der W Halogen, B $NHCOCH_3$ und R eine in der Kohlenhydratchemie gebräuchliche Schutzgruppe bedeuten, unter Walden-Umkehr am C-1-Atom des Zuckerrestes zu einem Per-O-substituierten $\beta$-Glycosid umzusetzen und von letzterem nach an sich bekannten Methoden die Hydroxy-Schutzgruppen abzuspalten.

Die Umsetzung der Verbindungen der Formel XVII und XIX in der zuvor genannten Bedeutung zu N-Acetyl-$\beta$-D-glucosaminiden der allgemeinen Formel II, wird vorzugsweise in Gegenwart von Säurefängern, wie Alkalihydroxid, - carbonat oder -bicarbonat in wässrigem Aceton oder unter Phasentransferbedingungen in einem Wasser/Benzol oder Wasser/Chloroform-Gemisch vorgenommen (vgl. Synthesis 223 (1988).

Des weiteren können die N-Acetyl-$\beta$-D-glucosaminide der allgemeinen Formel II hergestellt werden, indem man die Leukoazomethinfarbstoffe der allgemeinen Formel XVII mit A gleich Wasserstoff zunächst mit Alkalihydroxid oder -alkoholat in die Alkalisalze bzw. mittels gegebenenfalls substituierten Aminen in die Ammoniumsalze überführt, wobei Alkalimetall und Ammoniumion, die zuvor gegebene Bedeutung haben können und diese dann in dipolaren, aprotischen Lösungsmitteln wie Aceton, Dimethylsulfoxid Dichlormethan, oder Dimethylformamid mit den per-O-substituierten 1-Halogen-N-acetyl-glucosaminen umsetzt.

Ferner haben sich bei der Synthese von N-Acetylglucosaminiden der allgemeinen Formel II aus den Leukoazomethinfarbstoffen der allgemeinen Formel XVII und 1-Halogeno-N-acetylglucosaminen Zusätze von einzelnen Silbersalzen oder Gemischen von Silbersalzen (Silberoxid, - carbonat auf Celite®, -triflat, -salicylat) und/oder von einzelnen Quecksilbersalzen oder Gemischen von Quecksilbersalzen (Quecksilberbromid, -cyanid, -acetat, -oxid), gegebenenfalls unter Verwendung von Trocknungsmitteln wie Calciumchlorid oder Drierit®, in Lösungsmitteln, wie Methylenchlorid, Chloroform, Benzol, Toluol oder Dioxan, bewährt.

Ebenso kann ein Oxazolin der allgemeinen Formel XIX, in der B und W zusammen die Gruppe

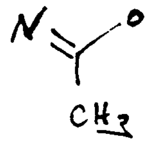

bedeuten, in Gegenwart von organischen Säuren wie z.B. p-Toluolsulfonsäuren oder Lewis-Säuren wie $BF_3$-Etherat oder $FeCl_3$ zur Synthese von N-Acetylglucosaminiden der allgemeinen Formel II verwendet werden. Beispiele für solche Glycosidierungsreaktionen sind in Carbohydrate Research 136, 309-323 (1985) und 64, 334-338 (1978) beschrieben.

Schließlich sind Verfahren zur Herstellung der N-Acetylglucosaminide der allgemeinen Formel II durchführbar, bei denen in der Substanz der allgemeinen Formel XIX B eine mit einer Schutzgruppe z.B. Benzyloxycarbonyl, Allyloxycarbonyl, Dichloracetamido oder Phthalimido substituierte Aminogruppe oder ein geeigneter unter den Glycosidierungsbedingungen stabiler Substituent, aus dem eine Aminogruppe freigesetzt werden kann z.B. ein Azidrest bedeutet. Es wird die Glycosidierungsreaktion durchgeführt, durch Abspaltung der Schutzgruppen nach den Methoden der Peptidchemie oder Reduktion einer Azidogruppe, die Aminogruppe freigesetzt, die in einem letzten Schritt selektiv N-acetyliert wird (siehe z.B. J. Org.Chem. 32, 3767 (1967)).

Die Abspaltung der Schutzgruppen erfolgt nach der in der Kohlehydratchemie bekannten Weise durch Hydrogenolyse bei Schutzgruppen auf Benzylbasis, durch Einwirkung von Natriummethylat, Natriumcyanid oder Natriumbicarbonat in Methanol zur Abspaltung von Acylgruppen z.B. Acetyl. Die Methoden der Schutzgruppenabspaltung sind beschrieben in Adv. Carbohydr. Chem. Biochem. 39, 13 (1981).

Die Synthese von 1-Halogeno-N-acetyl-glucosaminen ist beschrieben, z. B. in Org Synth. Vol. 46, S. 1, Methods in Carbohydrate Chem. 6, 282 (1972) oder J. Org. Chem. 26, 445 (1961).

Die zur Herstellung der erfindungsgemäßen Verbindungen der allgemeinen Formel II benötigten Leukoazomethinfarbstoffe der allgemeinen Formel XVII'

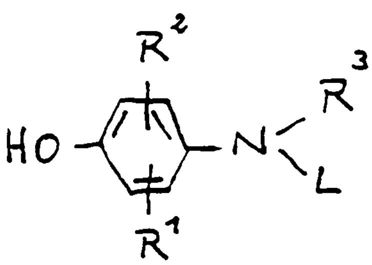

(XVII')

in der $R^1$-$R^3$ und L die für die allgemeine Formel II angegebene Bedeutung haben, wobei L vorzugsweise einen Pyrazoloheterocyclus-Rest der allgemeinen Formel IV

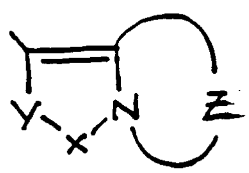

(IV)

darstellt, in der X-Y und Z die für entsprechenden Verbindungen der allgemeinen Formel II bereits angegebene Bedeutung haben, lassen sich durch Reduktion der entsprechenden Farbstoffe der allgemeinen Formel XX,

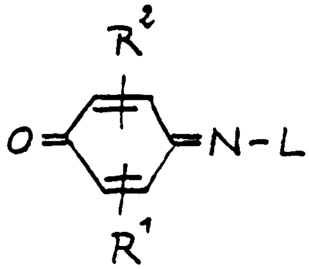

(XX)

in der
$R^1$, $R^2$ und L die für Verbindungen der allgemeinen Formel XVII' angegebene Bedeutung haben, nach an sich bekannten Methoden mit Reduktionsmitteln, wie katalytische Hydrierung, Natriumborhydrid, Palladium/Hydrazin oder Natriumdithionit herstellen. Solche Reduktionsmittel sind in Houben/Weyl Bd. 4/1c, und 4/1d beschrieben.

Acylgruppen $R^3$, wie sie für Verbindungen der allgemeinen Formel II angegeben sind, können entweder auf der Stufe des Leukofarbstoffs der allgemeinen Formel XVII' mit $R^3$ gleich Wasserstoff oder des geschützten per O-substituierten N-Acetyl-glucosaminids, wie es bei der Herstellung der Verbindungen der allgemeinen Formel II mit G gleich Glycosid vorkommt eingeführt werden. Es werden aktivierte Säurederivate z.B. Halogenide, Anhydride, gemischte Anhydride verwendet, wie sie aus der Peptidchemie bekannt sind.

Die für die Herstellung von Verbindungen der allgemeinen Formel XVII' erforderlichen Farbstoffe der allgemeinen Formel XX lassen sich am zweckmäßigsten durch oxidative Kupplung einer Aminoverbindung der allgemeinen Formel XXI

$NH_2$-L (XXI)

in der L die für die allgemeine Formel II angegebene Bedeutung hat, vorzugsweise aber einen Pyrazoloheterocyclusrest der allgemeinen Formel IV darstellt, in der X-Y und Z die für Verbindungen der allgemeinen Formel II angegebenen Bedeutungen haben mit einem Phenol der allgemeinen Formel XXII

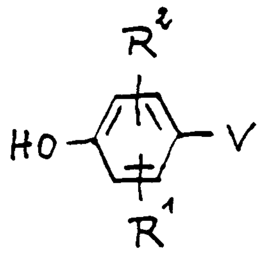

(XXII)

in der $R^1$ und $R^2$ die für Verbindungen der allgemeinen Formel II angegebenen Bedeutungen haben und V Wasserstoff, Halogen, Carboxy oder $SO_3H$ darstellt, herstellen. Dazu werden eine Aminoverbindung der allgemeinen Formel XXI und ein Phenol der allgemeinen Formel XXII bevorzugt mit V gleich Wasserstoff in Gegenwart eines Oxidationsmittels wie $K_3$ $[Fe(CN)_6]$, $K_2S_2O_8$, $KHSO_5$, Jod, $H_2O_2$/Peroxidase, Bleidioxid, NaOCl, NaOBr oder organischen Oxidationsmitteln, wie N- Bromsuccinimid oder verwandten Verbindungen umgesetzt.

Ferner lassen sich die Farbstoffe der allgemeinen Formel XX auch durch Umsetzung von N-Halogeniminen der allgemeinen Formel XXIII

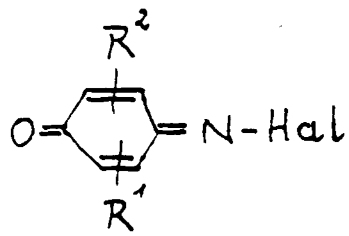

(XXIII)

in der $R^1$ und $R^2$ die für Verbindungen der allgemeinen Formel II angegebene Bedeutung haben und Hal einen Halogenrest bedeutet, wobei Halogen Fluor, Chlor, Brom oder Jod, vorzugsweise Chlor, ist, mit Verbindungen der allgemeinen Formel XXIV

L-H (XXIV)

in der L die für die allgemeine Formel II angegebene Bedeutung hat, vorzugsweise aber einen Pyrazoloheterocyclus-Rest der allgemeinen Formel IV bedeutet, wobei X-Y und Z die für Verbindungen der allgemeinen Formel II angegebene Bedeutung haben, herstellen.

Die Reaktionsbedingungen lassen sich analog den in Houben/Weyl Bd. 7/3 b S. 296 f. beschriebenen wählen.

Für die Aminoverbindungen der allgemeinen Formel XXI gibt es folgende Herstellungsmethoden. Wenn L einen Rest der allgemeinen Formel III bedeutet, wie er für Verbindungen der allgemeinen Formel II definiert ist, sind diese Aminoverbindungen bekannt oder können auf analoge Weise wie bekannte hergestellt werden. Üblicherweise geht man von entsprechend N,N-disubstituierten Anilinen aus, die nitrosiert werden. Reduktion der Nitrosogruppe liefert die p-Phenylendiaminderivate der allgemeinen Struktur $L-NH_2$ (siehe J. Amer. Chem. Soc. 73, 3100 (1951)).

Verbindungen der allgemeinen Formel XXI, worin L einen Pyrazoloheterocyclusrest der allgemeinen Formel IV bedeutet, können analog bekannten Methoden hergestellt werden, indem man eine Verbindung der allgemeinen Formel XXIV, in der L einen Pyrazoloheterocyclus-Rest der allgemeinen Formel IV bedeutet nach an sich bekannten Methoden in die entsprechende Aminoverbindung überführt.

Dies kann

a) durch Umsetzung mit Salpetersäure oder Salpetersäure im Gemisch mit Schwefelsäure und/oder Acetanhydrid zu der entsprechenden Nitro-Verbindung oder

b) durch Reaktion mit salpetriger Säure zu der entsprechenden Nitrosoverbindung oder

c) durch Reaktion mit einem aromatischen Diazoniumsalz zu der entsprechenden Arylazoverbindung

und anschließende Reduktion erreicht werden.

Nitro-, Nitroso- und Arylazoreste, das sind Reste

Aryl-N=N-, wobei Aryl die gleiche Bedeutung haben kann, wie bereits für Arylreste und andere solche Reste enthaltende Gruppen erläutert, lassen sich durch Reduktion mit Reagentien, wie Zink in Säure, beispielsweise Salzsäure oder Eisessig, Natriumdithionit, Zinn in Säure, beispielsweise Salzsäure, Zinn(II)-chlorid oder durch katalytische Hydrierung beispielsweise über Palladium/Kohle in die Aminoverbindung überführen. Solche Reaktionen sind in Houben-Weyl, Methoden der organischen Chemie, Bd. 11/1, S. 341 f beschrieben.

Die Einführung von Nitro, Nitroso oder Arylazogruppen ausgehend von Verbindungen der Formel XXIV kann durch Nitrierung mit Salpetersäure oder Salpetersäure in Gemischen mit konzentrierter Schwefelsäure oder Acetanhydrid erfolgen.

Durch Nitrosierung mit salpetriger Säure oder durch Azokupplung mit aromatischen Diazoniumsalzen, lassen sich die Nitrosogruppe bzw. eine Arylazogruppe einführen. Beispiele für solche Reaktionen sind in Houben-Weyl, Methoden der organischen Chemie Bd. 10/1 und 10/3 beschrieben.

Liegen Heterocyclen der allgemeinen Formel XXIV vor, in der H nicht Wasserstoff, sondern Carboxyl, Alkoxycarbonyl oder Alkylcarbonyl bedeutet, so können diese durch Hydrolyse mit konzentrierter Salzsäure und - bei Carbonsäuren - thermischer Decarboxylierung in die Verbindungen der allgemeinen Formel XXII überführt werden. Daran schließt sich dann die Einführung einer Nitro-, Nitroso- oder Arylazogruppe an.

Stickstoffatome, die nicht an einer Doppelbindung stehen, und in den Resten X-Y oder Z der allgemeinen Formel IV vorkommen, können gegebenenfalls alkyliert oder aralkyliert sein. Die N-Alkylierung bzw. N-Aralkylierung läßt sich durch Umsetzung der entsprechenden Verbindungen der Formel XXIV, vorzugsweise jedoch solchen Heterocyclen, in denen H nicht Wasserstoff, sondern Nitro, Nitroso, Alkoxycarbonyl, Acyl oder Arylazo bedeutet, mit Alkylierungs- bzw. Aralkylierungsmitteln, wie Alkyl- oder

Aralkylhalogeniden, Dialkyl- bzw. Diaralkylsulfaten oder Arylsulfonsäurealkylestern bzw. -aralkylestern in Gegenwart einer Base, wie Natriumhydrid, tertiären Aminen, Alkalicarbonaten oder Natriumhydroxid in Lösungsmitteln, wie Dimethylformamid oder wässrig-alkoholische Systeme durchführen.

Die benötigten Ausgangsprodukte der allgemeinen Formel XXIV oder solche der allgemeinen Formel XXIV entsprechende Verbindungen, in denen H ersetzt ist durch Alkoxycarbonyl oder Acyl, wobei L einen Pyrazoloheterocyclen-Rest der allgemeinen Formel IV bedeutet, sind beschrieben oder lassen sich analog zu bekannten Verbindungen synthetisieren. Informationen über die Herstellung der heterocyclischen Systeme sind in folgenden Publikationen enthalten: G.P. Ellis "Synthesis of fused Heterocyles", in "The Chemistry of Heterocyclic Compounds", E.C. Taylor Eds., 1987, John Wiley and Sons; P.N. Preston, "Condensed Imidazoles" in "The Chemistry of Heterocyclic Compounds"` A. Weissberger und E.C. Taylor Eds., 1986, John Wiley and Sons; Adv. of Het. Chem. 36, 343 (1984); Chem. Pharm. Bull. 22, 482 (1974); J. Het. Chem. 12, 481 (1975); Chem. Pharm. Bull. 22, 1814 (1974); Ann. 660, 104 (1962); Chem. Pharm. Bull. 23, 452 (1975); J. Het. Chem. 10, 411 (1973); J. Chem. Soc. Perkin I 2047 (1977).

Die folgenden Beispiele sollen die Erfindung weiter erläutern, ohne sie hierauf zu beschränken.

Beispiel 1

1.1 (4-Hydroxyphenyl)-(2-methylpyrazolo-[1,5-a]-pyridin-3-yl)-amin

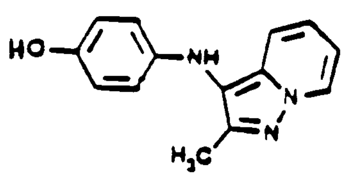

1.1.1 7 g 3-Acetyl-2-methyl-pyrazolo-[1.5-a]-pyridin (J. Org. Chem. 42, 443 (1977)) werden in 140 ml 6 N Salzsäure gelöst und bei 0 °C tropfenweise mit einer Lösung von 5,52 g Natriumnitrit in Wasser versetzt. Nach 2 Stunden wird das Eisbad entfernt, die Reaktionsmischung über Nacht bei Raumtemperatur stehen gelassen und dann auf pH 9 gestellt. Die ausgefallene Nitrosoverbindung (6.4 g) wird abgesaugt und in ca. 150 ml 2 N Salzsäure gelöst. Die Lösung der Nitrosoverbindung wird mit 15 g Zinn (II)chlorid-Dihydrat versetzt und 1 Stunde bei Raumtemperatur gerührt. Zur Vervollständigung der Reduktion werden dann noch einmal 6 g Zinn (II)chlorid-Dihydrat in 20 ml konzentrierter Salzsäure zugegeben. Das Rohprodukt wird an Kieselgel mit Essigsäureethylester chromatographiert. Die Produktfraktionen werden eingeengt, in Ethanol gelöst und mit ethanolischer Salzsäure versetzt. Der nach einiger Zeit ausfallende Niederschlag wird abgesaugt und getrocknet. Man erhält 3.1 g (45 % der Theorie) 3-Amino-2-methyl-pyrazolo-[1,5-a]-pyridin-hydrochlorid mit Fp. > 275 °C, $R_f$ (Kieselgel, Essigsäureethylester/Aceton/ Eisessig/Wasser
50:25:12,5:12,5) = 0.6.

1.1.2 2,82 g Phenol werden in 75 ml Pyridin gelöst und die Lösung mit 450 ml Wasser versetzt. Dann fügt man eine Lösung von 5,5 g 3-Amino-2-methyl-pyrazolo-[1,5-a]-pyridin-hydrochlorid aus 1.1.1 in 150 ml Wasser hinzu und versetzt die Mischung anschließend unter Rühren mit einer Lösung von 78 g Kaliumferricyanid in 450 ml Wasser. Der ausgefallene blaue Farbstoff wird abgesaugt, mit Wasser gewaschen und getrocknet. Ausbeute 4,85 g.
DC (Kieselgel, Essigsäureethylester/Methylenchlorid 1:1): $R_f$ = 0,5).

1.1.3 Zur Reduktion zum Leukofarbstoff wird der Farbstoff in 200 ml Essigsäureethylester gelöst und die Lösung mit ca. 100 ml gesättigter Sodalösung versetzt. Die Lösung wird bis zur Entfärbung unter kräftigem Schütteln mit Natriumdithionit versetzt. Die organische Phase wird abgetrennt, getrocknet und bis auf ein kleines Volumen eingeengt. Man versetzt mit Ligroin und filtriert den ausgefallenen Niederschlag ab, der mit Ligroin gewaschen und anschließend getrocknet wird. Man erhält 4,45 g der Titelverbindung, die für die weitere Verarbeitung rein genug ist. Eine Reinigung ist durch Chromatographie über Kieselgel mit Methylenchlorid/Methanol (98:2) möglich.
$R_f$ (Kieselgel, Essigsäureethylester/Methylenchlorid (1:1)) = 0,6
$R_f$ (Kieselgel, Methylenchlorid/Methanol (95:5)) = 0,3

1.2 N-Acetyl-β-D-glucose-2-aminid von (4-Hydroxyphenyl)-(2-methylpyrazolo-[1,5-a]-pyridin-3-yl)-amin

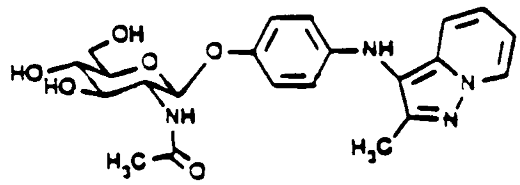

1.2.1 2,6 g des in 1.1 erhaltenen Leukofarbstoffes, 8,83 g 2-Acetamido-3,4,6-tri-0-acetyl-2-deoxy-α-D-glucosylchlorid und 4,21 g Benzyltriethylammoniumbromid werden zu einem Gemisch von 125 ml Chloroform und 125 ml Wasser gegeben. Die Mischung wird heftig gerührt, mit 8,7 g Kaliumcarbonat versetzt und 6 Stunden unter Rückfluß gekocht, wobei nach 3 Stunden nochmals 4,4 g der Halogenose und 4,3 g Kaliumcarbonat zugegeben wird. Die Chloroformphase wird abgetrennt, mit Natriumsulfat getrocknet und eingeengt. Der Rückstand wird an Kieselgel mit Essigsäureethylester chromatographiert. Die produkthaltigen Fraktionen werden eingeengt.

1.2.2 460 mg des in 1.2.1 erhaltenen geschützten Zuckerderivates werden in 10 ml Methanol gelöst, mit 0,9 g Natriumbicarbonat versetzt und 3 Stunden bei Raumtemperatur kräftig gerührt. Die Reaktionsmischung wird filtriert, das Filtrat eingedampft und der Rückstand an Kieselgel mit Essigsäureethylester/Methanol (8:2) chromatographiert. Die produkthaltigen Fraktionen werden vereinigt und eingedampft. Der Rückstand wird in wenig Methanol aufgenommen und die Lösung mit Ether versetzt.

Der so entstehende Niederschlag wird abgesaugt und mit Ether gewaschen. Man erhält die Titelverbindung mit Fp 188-191 °C; Rf (Kieselgel, Toluol/Essigsäure/Essigsäureethylester/Methanol = 1:1:1) = 0,35.

Beispiel 2

2.1 (4-Hydroxyphenyl)- (pyrazolo- [1,5-a]-pyridin-3-yl)-amin

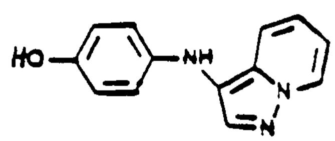

2.1.1 2 g Pyrazolo-[1.5-a]-pyridin (Ann. Chem. 498 (1977)) werden in 6 N Salzsäure (30 ml) gelöst, die Lösung auf 0 °C gekühlt und dazu langsam eine Lösung von 6.9 g Natriumnitrit in 30 ml Wasser getropft. Nach 1 Stunde ist die Nitrosierung beendet. Man setzt ca. 100 ml Wasser zu und extrahiert mehrfach mit Essigsäureethylester. Die organische Phase wird getrocknet und eingeengt. Man erhält 9.6 g 3-Nitroso-pyrazolo-[1.5-a]-pyridin.

2.1.2 9 g der nach 2.1.1 erhaltenen Nitrosoverbindung werden in eine Lösung von 22 g Zinn (II)-chlorid-Dihydrat in 180 ml konzentrierter Salzsäure eingetragen. Die Reaktionsmischung wird 1 Stunde bei Raumtemperatur gerührt und zur Vervollständigung der Reduktion mit 8 g Zinn (II)-chlorid Dihydrat in 30 ml konzentrierter Salzsäure versetzt.

Die Suspension wird auf ca. 150 g Eis gegossen, mit Natriumhydroxid auf pH 12 gestellt und rasch mit Essigsäureethylester extrahiert. Die Essigesterphase wird getrocknet und eingeengt. Der Rückstand wird in ca. 350 ml Ether gelöst und mit etherischer Chlorwasserstoffsäure versetzt. Der ausgefallene Niederschlag wird abfiltriert, mit Ether gewaschen und getrocknet. Man erhält 11.3 g (100 % der Theorie) 3-Amino-pyrazolo-[1,5-a]-pyridin-hydrochlorid mit Fp. 228-232 ° C

$R_f$ (Kieselgel, Essigsäureethylester/Methanol 9:1) = 0,52

2.1.3 Der Leukofarbstoff (4-Hydroxyphenyl)-(pyrazolo-[1,5-a]-pyridin-3-yl)-amin wird analog 1.1.2 und 1.1.3 unter Verwendung von 3-Amino-pyrazolo-[1,5-a]-pyridin als Ausgangsmaterial erhalten

$R_f$ (Kieselgel, Essigsäureethylester/Ether (1:1)) = 0,68.

2.2 N-Acetyl-β-D-glucose-2-aminid von (4-Hydroxyphenyl)-(pyrazolo-[1,5-a]-pyridin-3-yl)-amin

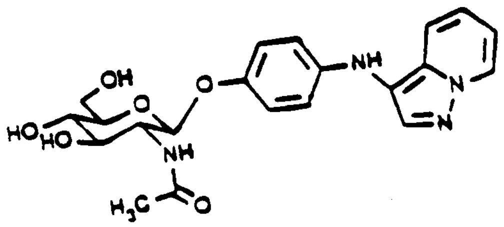

Aus dem in 2.1 erhaltenen Leukofarbstoff und 2-Acetamido-3,4,6-tri-0-acetyl-2-deoxy-α -D-glucosylchlorid erhält man analog Beispiel 1.2 die Titelverbindung mit Fp 143 °C; Rf (Kieselgel, Toluol/Essigsäureethylester/Methanol = 2:1:1) = 0,27.

Beispiel 3

a) Ein Filterpapier der Firma Schleicher und Schüll (23 SL) wird nacheinander mit folgenden Lösungen imprägniert und getrocknet.

| | | |
|---|---|---|
| 1. | Citratpuffer<br>Kaliumjodat | 200 mMol/l, pH 5,0<br>20 mMol/l |
| 2. | Phenylsemicarbazid<br>Hydrolase-Substrat aus Beispiel 1 in Methanol | 3 mMol/l<br>10 mMol/l |

b) Tränkung wie in Beispiel 3 a, als Stabilisator wird jedoch Methyl-phenylsemicarbazid (1,5 mMol/l) verwendet.

Taucht man die Testpapiere aus Beispielen 3 a) und 3 b) in Harn, der keine N-Acetyl-β-D-glucosaminidase (β-NAGase) enthält, oder tropft Harn auf das Testpapier, so färbt sich der Test über 10 Minuten nicht. Enthält der Harn eine β-NAGase-Konzentration von etwa 20 U/l, so erhält man nach ca. 5 Minuten eine deutliche Verfärbung des Testpapiers.

Fertigt man Teststreifen analog der angegebenen Beispiele 3 a) und 3 b) ohne Stabilisator, so erhält man anfangs vergleichbare Ergebnisse, aber nach Lagerung von einer Woche bei 45 °C sind die Teststreifen rot gefärbt. Stabilisierte Teststreifen sind nach dieser Zeit mit neu gefertigten Streifen vergleichbar.

Beispiel 4

a) Ein Filterpapier der Firma Schleicher und Schüll (23 SL) wird nacheinander mit folgenden Lösungen imprägniert und getrocknet:

| | | |
|---|---|---|
| 1. | Citratpuffer<br>Kaliumjodat | 100 mMol/l, pH 5,0<br>20 mMol/l |
| 2. | Phenylsemicarbazid<br>Hydrolase-Substrat aus Beispiel 2 in Methanol | 10 mMol/l<br>10 mMol/l |

b) Tränkung wie in Beispiel 4 a), als Stabilisator wird jedoch Methyl-phenylsemicarbazid verwendet.

Taucht man die Testpapiere aus Beispielen 4 a) und 4 b) in Harn, der keine β-NAGase enthält, oder tropft Harn auf das Testpapier, so färbt sich der Test über 10 Minuten nicht. Enthält der Harn eine β-NAGase-Konzentration von etwa 20 U/l, so erhält man nach ca. 5 Minuten eine deutliche Verfärbung des Testpapiers.

Fertigt man Teststreifen analog der angegebenen Beispiele 4 a) und 4 b) ohne Stabilisator, so erhält man anfangs vergleichbare Ergebnisse, aber nach Lagerung von einer Woche bei 45 °C sind die Teststreifen rot gefärbt. Stabilisierte Teststreifen sind nach dieser Zeit mit neu gefertigten Streifen

vergleichbar.

Beispiel 5

Ein analoges Ergebnis wie in Beispielen 3 und 4 wird auch erzielt, wenn ein Teststreifen gemäß Fig. 2 hergestellt wird:

Das Reagenzpapier (3) (Filterpapier 23 SL der Firma Schleicher und Schüll) wird

a) analog Beispiel 3 a
b) analog Beispiel 3 b
c) analog Beispiel 4 a
d) analog Beispiel 4 b

jedoch ohne Kaliumjodat imprägniert.

Das Netz (7) (NY 75 HC der Züricher Beuteltuchfabrik, Zürich, Schweiz, mit einer Fadendicke von 60 µm) wird mit einer wässrigen Lösung von Kaliumjodat (40 mMol/l) imprägniert und getrocknet. Reagenzpapier (3) und Netz (7), jeweils 6 mm x 6 mm groß, werden mit einem Abdecknetz (4) aus dem gleichen Material wie Netz (7) so auf einer steifen Polystyrolfolie (100 mm x 6 mm) mittels Schmelzkleber (5) fixiert, daß sich das Oxidationsnetz (7) zwischen Reagenzpapier (3) und Abdecknetz (4) befindet.

Taucht man die so hergestellten Teststreifen in Harn, der keine β-NAGase enthält, oder tropft Harn auf das Abdecknetz (4) auf, so färbt sich der Teststreifen über 10 Minuten nicht. Enthält der Harn eine β-NAGase-Konzentration von etwa 20 U/l, so erhält man nach ca. 5 Minuten eine deutliche Verfärbung des Reagenzpapiers.

Fertigt man Teststreifen wie oben beschrieben, aber ohne Stabilisator, so erhält man anfangs vergleichbare Ergebnisse, aber nach Lagerung von einer Woche bei 45 °C sind die Teststreifen rot verfärbt. Stabilisierte Teststreifen sind nach dieser Zeit mit neu gefertigten Streifen vergleichbar.

Beispiel 6

Es wird ein Testträger gemäß Fig. 2 analog Beispiel 5 hergestellt, wobei das Hydrolase-Substrat aus Beispiel 2 verwendet wird. Die Vortränkung des Reagenzpapiers erfolgt jedoch mit einer Ascorbinsäurehaltigen Lösung.

Das Reagenzpapier (3) wird nacheinander mit folgenden Lösungen getränkt und getrocknet:

| | | |
|---|---|---|
| 1. | Citratpuffer<br>Ascorbinsäure | 200 mMol/l, pH 5,0<br>3 mMol/l |
| 2. | Phenylsemicarbazid<br>Hydrolase-Substrat aus Beispiel 2 in Methanol | 0,5 mMol/l<br>10 mMol/l |

Das Oxidationsnetz (7) wird imprägniert mit einer wässrigen Lösung aus Kaliumjodat (40 mMol/l).

Auch hier wird bei Verwendung des erfindungsgemäßen Stabilisators eine Stabilisierung des Hydrolase-Substrats auf dem Testträger gegenüber solchen Teststreifen ohne Stabilisator festgestellt.

Durch Zugabe von Ascorbinsäure in das Reagenzpapier (3) kann die zur Stabilisierung notwendige Konzentration an Semicarbazid deutlich verringert werden. Man erhält so außerdem ein schnelleres Testsystem.

Ein vergleichbares Ergebnis wird auch erhalten, wenn Ascorbinsäure durch Natriumthiosulfat ersetzt wird.

Beispiel 7

Ein Papier der Firma Schleicher und Schüll (23 SL) wird in der folgenden Weise imprägniert:

1. Vortränkung durch 65 g Borsäure und 21 g Natriumhydroxid in 1 l Wasser, eingestellt mit 1 N Salzsäure auf pH 8,0.
2. Haupttränkung des vorgetränkten Vlieses mit einer Lösung, die in 1 1 Ethanol 0,78 g 3-(N-Toluol-4-sulfonyl-L-alanyl oxy)-indol (hergestellt gemäß EP-B-0 012 957, Beispiel 7, 21,7 ml Decanol und 10 mMol/l Phenylsemicarbazid enthält.

Tropft man auf dieses Reagenzpapier eine Lösung von Leukozyten, so verfärbt sich das Papier blau.

Fertigt man Testpapiere analog der oben beschriebenen Rezeptur aber ohne den Stabilisator, so erhält man anfangs vergleichbare Ergebnisse. Bei einer Belastungszeit von 3 Wochen bei 45° C färbt sich das

Papier aber bläulich. Papiere mit Stabilisator bleiben weiß.

Beispiel 8

Ein Papier der Firma Schleicher und Schüll (23 SL) wird wie folgt imprägniert:

1. Vortränkung durch Hepespuffer 50 mMol/L pH 7,5
2. Haupttränkung von 1 mMol/l

5-Bromo-4-chloro-3-indolyl-β-D-Galaktopyranosid (Firma Sigma Chemie GmbH, Deisenhofen, Deutschland) und 10 mMol/l Phenylsemicarbazid in Methanol.

Tropft man β-D-Galaktosidase-Lösung auf das Papier, so erhält man eine Blaufärbung. Fertigt man Testpapiere ohne den Stabilisator, so sind die Ergebnisse anfangs vergleichbar. Bei einer Belastungszeit von 3 Wochen bei 45° C färbt sich das Papier aber bläulich. Papiere mit Stabilisator bleiben weiß.

**Patentansprüche**

**1.** Verwendung eines 1-Arylsemicarbazides der allgemeinen Formel I

$Ar-NH-NH-CONH_2$ (I)

in der

Ar einen gegebenenfalls durch Alkyl, Alkoxy oder Halogen substituierten Arylrest bedeutet,

zur Stabilisierung eines Enzymsubstrats, aus dem durch enzymatische Hydrolyse ein Leukofarbstoff gebildet werden kann, der seinerseits durch ein Oxidationsmittel zu einem Farbstoff umgesetzt werden kann.

**2.** Verwendung eines 1-Arylsemicarbazides gemäß Anspruch 1 in Gegenwart eines Oxidationsmittels.

**3.** Verwendung eines 1-Arylsemicarbazides gemäß einem der Ansprüche 1 oder 2, dadurch gekennzeichnet, daß das Enzymsubstrat ein N- und O-substituiertes Aminophenolderivat der allgemeinen Formel II

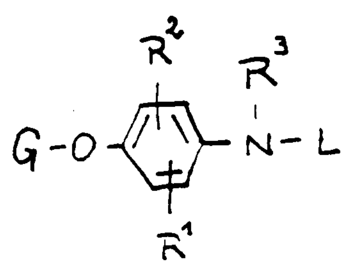

ist, in der

G einen organischen oder anorganischen Säurerest oder einen Glycosidrest darstellt,

$R^1$ und $R^2$, die gleich oder verschieden sind, Wasserstoff, Halogen, $SO_3H$, $PO_3H_2$ oder ein Salz dieser Säuregruppe, eine Hydroxy-, Nitro-, Carboxy-, Carboxamido- oder Cyanogruppe oder eine gegebenenfalls durch einen oder mehrere Hydroxy-, Carboxy-, Halogen-, Cyano-, $SO_3H$- oder $PO_3H_2$-Reste oder ein Salz eines dieser Säurereste substituierte Alkyl-, Alkenyl-, Alkoxy-, Alkylsulfinyl-, Alkylsulfonyl-, Alkoxycarbonyl-, Alkylcarbonyl-, Aryl-oder Aralkylgruppe bedeuten oder wenn sich beide Reste an benachbarten Kohlenstoffatomen befinden gemeinsam eine 1,4-Butadiendiylgruppe darstellen, die gegebenenfalls ein-oder mehrmals durch $SO_3H$, $PO_3H_2$ oder ein Salz dieser Säuregruppen, eine Alkyl- oder/und eine Carboxygruppe substituiert ist,

$R^3$ Wasserstoff, CO-COOH, $SO_3H$, $PO_3H_2$ oder ein Salz dieser Säuregruppen, eine gegebenenfalls ein- oder mehrfach durch Halogen, $CO_2H$, $SO_3H$ oder/und $PO_3H_2$ oder ein Salz dieser Säuregruppen substituierte Alkylcarbonylgruppe oder eine gegebenenfalls ein- oder mehrfach durch $SO_3H$, $PO_3H_2$ oder ein Salz dieser Säurereste substituierte Arylcarbonylgruppe ist und

L einen Rest der allgemeinen Formel III

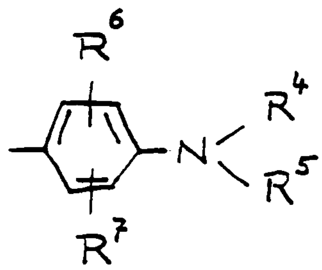

wobei

$R^4$ und $R^5$, die gleich oder verschieden sein können, Alkyl bedeuten oder zusammen eine gesättigte Kohlenwasserstoffkette mit 3 - 6 Gliedern darstellen, die durch Sauerstoff, Schwefel oder Stickstoff unterbrochen sein kann, wobei Alkyl bzw. die Kohlenwasserstoffkette gegebenenfalls ein- oder mehrfach durch eine Hydroxy-, Carboxy-, Alkoxycarbonyl-, Alkoxy-, $SO_3H$- oder $PO_3H_2$-Gruppe, ein Salz eines dieser Säurereste oder Halogen substituiert ist und

$R^6$ und $R^7$, die gleich oder verschieden sein können, Wasserstoff, Halogen, eine Hydroxy- oder Carboxamidogruppe oder eine gegebenenfalls durch einen oder mehrere Hydroxy-, Carboxy-, Halogen-, $SO_3H$- oder $PO_3H_2$-Reste oder ein Salz eines dieser Säurereste substituierte Alkyl-, Alkoxy-, Alkylcarbonyl-, Alkoxycarbonyl-, Aryl- oder Aralkylgruppe bedeutet oder

L einen Pyrazoloheterocyclus-Rest der allgemeinen Formel IV

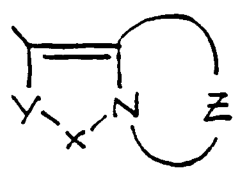

darstellt,

in der

X-Y $NR^8$-CO oder $N=CR^9$ bedeutet,

wobei

$R^8$ Wasserstoff oder Alkyl und

$R^9$ Alkyl, Alkenyl, Alkoxy, Alkylthio, Aryl, Aralkyl, gegebenenfalls jeweils substituiert durch Hydroxy, Dialkylphosphinyl,

Carboxy, $SO_3H$, $PO_3H_2$, ein Salz eines dieser Säurereste oder/und Alkoxycarbonyl; Amino, das gegebenenfalls durch ein oder zwei gegebenenfalls einen oder mehrere Hydroxy-, Carboxy- oder/und Alkoxycarbonylreste tragenden Alkylrest substituiert ist wobei, wenn Amino durch 2 Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann, oder Amino gegebenenfalls durch ein oder zwei Acylgruppen, Alkoxy- oder/und Aralkoxy-Carbonylgruppen, $H_2N$-CO, Alkyl-, Aralkyl- oder/und Arylcaramoylgruppen substituiert ist; oder

Wasserstoff, Carboxy, Alkoxycarbonyl, Carboxamido oder Halogen ist, und

Z $NR^{10}$ -N=N bedeutet

wobei $R^{10}$ Wasserstoff, Alkyl oder Aralkyl ist oder

Z eine ungesättigte Kette mit 3 bis 5 Gliedern aus Stickstoffatomen oder aus Kohlenstoffatomen und gegebenenfalls einem oder mehreren Stickstoff- oder Schwefelatomen darstellt, wobei Kohlenstoffatome gegebenenfalls durch Alkyl, Alkoxy, Hydroxyalkyl, Alkylthio, Hydroxy, Aralkyl, Aryl, Carboxy, Carboxamido, Alkoxycarbonyl, Cyano, Amino, das gegebenenfalls durch ein oder zwei gegebenenfalls einen oder mehrere Hydroxy-, Carboxy- oder/und Alkoxycarbonylreste tragenden Alkylrest substituiert ist, oder/und Halogen sowie Stickstoffatome, die nicht über eine Doppelbindung gebunden sind, gegebenenfalls durch Alkyl oder Aralkyl substituiert sind oder zwei benachbarte Kettensubstituenten gegebenenfalls eine Alkylengruppe bilden, die ihrerseits gegebenenfalls mit Aryl substituiert oder anelliert ist oder ein entsprechender tautomerer Rest.

**4.** Verwendung eines 1-Arylsemicarbazides gemäß Anspruch 3, dadurch gekennzeichnet, daß G einen Galactosid-, Glucosid-, Mannosid-, N-Acetyl-glucosaminid- oder einen Oligosaccharidrest mit 2 - 10 Monosaccharid-Einheiten bedeutet.

**5.** Verwendung eines 1-Arylsemicarbazides gemäß Anspruch 3, dadurch gekennzeichnet, daß G einen N-Acetyl-$\beta$-D-glucosaminidylrest darstellt.

**6.** Verwendung eines 1-Arylsemicarbazides gemäß Anspruch 3, dadurch gekennzeichnet, daß
G $PO_3MM'$, $SO_3M$, einen carboxygebundenen Alkancarbonsäure-, Aminosäure- oder Oligopeptidrest und
M und M' Wasserstoff, ein Alkali-, Erdalkali- oder Ammoniumion bedeuten.

**7.** Verfahren zur Stabilisierung eines Enzymsubstrats, aus dem durch enzymatische Hydrolyse ein Leukofarbstoff gebildet werden kann, der seinerseits durch ein Oxidationsmittel zu einem Farbstoff umgesetzt werden kann, dadurch gekennzeichnet, daß das Substrat in Kontakt mit einem 1-Arylsemicarbazid der allgemeinen Formel I

$Ar\text{-}NH\text{-}NH\text{-}CONH_2$ (I)

in der
Ar einen gegebenenfalls durch Alkyl, Alkoxy oder Halogen substituierten Arylrest bedeutet,
gebracht wird.

**8.** Diagnostisches Mittel zur kolorimetrischen Bestimmung eines Enzyms enthaltend ein Enzymsubstrat aus dem durch enzymatische Hydrolyse ein Leukofarbstoff gebildet werden kann, der seinerseits durch ein Oxidationsmittel zu einem Farbstoff umgesetzt werden kann, dadurch gekennzeichnet, daß es zur Stabilisierung des Enzymsubstrats ein 1-Arylsemicarbazid der allgemeinen Formel I

$Ar\text{-}NH\text{-}NH\text{-}CONH_2$ (I)

in der
Ar einen gegebenenfalls durch Alkyl, Alkoxy oder Halogen substituierten Arylrest bedeutet,
enthält.

**9.** Diagnostisches Mittel gemäß Anspruch 8, dadurch ge-kennzeichnet, daß es zusätzlich ein Oxidationsmittel enthält

**10.** Diagnostisches Mittel gemäß einem der Ansprüche 8 oder 9, dadurch gekennzeichnet, daß es die Komponenten in trägergebundener Form enthält.

**Claims**

**1.** Use of a 1-arylsemicarbazide of the general formula I

$Ar\text{-}NH\text{-}NH\text{-}CONH_2$ (I)

in which Ar signifies an aryl radical possibly substituted by alkyl, alkoxy or halogen, for the stabilisation of an enzyme substrate from which, by enzymatic hydrolysis, a leuko compound can be formed which, in turn, can be reacted by an oxidation agent to give a coloured material.

2. Use of a 1-arylsemicarbazide according to claim 1 in the presence of an oxidation agent.

3. Use of a 1-arylsemicarbazide according to one of claims 1 or 2, characterised in that the enzyme substrate is an N- or O-substituted aminophenol derivative of the general formula II

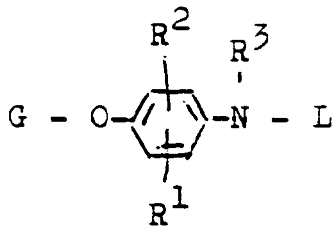

in which G represents an organic or inorganic acid residue or a glycoside residue, $R^1$ and $R^2$, which are the same or different, signify hydrogen, halogen, $SO_3H$, $PO_3H_2$ or a salt of this acid group, a hydroxyl, nitro, carboxyl, carboxamido or cyano group or an alkyl, alkenyl, alkoxy, alkylsulphinyl, alkylsulphonyl, alkoxycarbonyl, alkylcarbonyl, aryl or aralkyl group possibly substituted by one or more hydroxyl, carboxyl, halogen, cyano, $SO_3H$ or $PO_3H_2$ radicals or a salt of these acid residues or, when both substituents are present on neighbouring carbon atoms, together represent a 1,4-butadiendiyl group which is possibly substituted one or more times by $SO_3H$, $PO_3H_2$ or a salt of these acid groups, an alkyl and/or a carboxyl group, $R^3$ is hydrogen, -CO-COOH, $SO_3H$, $PO_3H_2$ or a salt of these acid groups, an alkylcarbonyl group group possibly substituted one or more times by halogen, $CO_2H$, $SO_3H$ and/or $PO_3H_2$ or a salt of these acid groups or an arylcarbonyl group possibly substituted one or more times by $SO_3H$, $PO_3H_2$ or a salt of these acid radicals and L is a radical of the general formula III

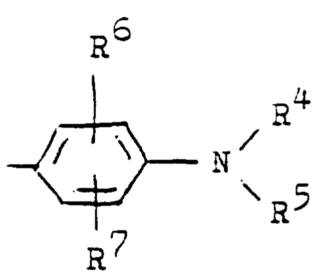

(III)

whereby $R^4$ and $R^5$, which can be the same or different, signify alkyl or together represent a saturated hydrocarbon chain with 3 - 6 members which can be interrupted by oxygen, sulphur or nitrogen, whereby alkyl or the hydrocarbon chain is possibly substituted one or more times by hydroxyl, carboxyl, alkoxycarbonyl, alkoxy, $SO_3H$ or $PO_3H_2$ groups, a salt of one of these acid radicals or halogen and $R^6$ and $R^7$, which can be the same or different, signify hydrogen, halogen, a hydroxyl or carboxamido group or an alkyl, alkoxy, alkylcarbonyl, alkoxycarbonyl, aryl or aralkyl group possibly substituted by one or more hydroxyl, carboxyl, halogen, $SO_3H$ or $PO_3H_2$ radicals or a salt of these acid radicals or L represents a pyrazolo-heterocyclic radical of the general formula IV

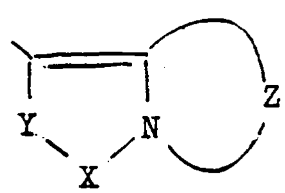

(IV)

in which X-Y signifies $NR^8$-CO or $N=CR^9$, whereby $R^8$ is hydrogen or alkyl and $R^9$ alkyl, alkenyl, alkoxy, alkylthio, aryl, aralkyl, possibly in each case substituted by hydroxyl, dialkylphosphinyl, carboxyl, $SO_3H$, $PO_3H_2$, a salt of these acid radicals and/or alkoxycarbonyl; amino which is possibly substituted by one or two alkyl radicals possibly substituted by one or more hydroxyl, carboxyl and/or

alkoxycarbonyl radicals, whereby, when amino is substituted by 2 alkyl radicals, these radicals can also be joined to form a ring which, apart from the N-atom of the amino group, can possibly also be interrupted by oxygen, sulphur or a further nitrogen atom or amino possibly substituted by one or two acyl groups, alkoxy- and/or aralkoxycarbonyl groups, $H_2N$-CO, alkyl, aralkyl and/or arylcarbamoyl groups; or is hydrogen, carboxyl, alkoxycarbonyl, carboxamido or halogen and Z signifies $NR^{10}$-N=N, whereby $R^{10}$ is hydrogen, alkyl or aralkyl or Z represents an unsaturated chain with 3 to 5 members of nitrogen atoms or of carbon atoms and possibly one or more nitrogen or sulphur atoms, whereby carbon atoms are possibly substituted by alkyl, alkoxy, hydroxyalkyl, alkylthio, hydroxyl, aralkyl, aryl, carboxyl, carboxamido, alkoxycarbonyl, cyano, amino, which is possibly substituted by one or two alkyl radicals possibly carrying one or more hydroxyl, carboxyl and/or alkoxycarbonyl radicals, and/or halogen, as well as nitrogen atoms, which are not connected via a double bond, possibly substituted by alkyl or aralkyl or two neighbouring chain substituents possibly form an alkylene group which, in turn, is possibly substituted or anellated with aryl or a corresponding tautomeric radical.

**4.** Use of a 1-arylsemicarbazide according to claim 3, characterised in that G signifies a galactoside, glucoside, mannoside, N-acetylglucosaminide or oligosaccharide radical with 2 - 10 monosaccharide units.

**5.** Use of a 1-arylsemicarbazide according to claim 3, characterised in that G represents an N-acetyl-$\beta$-D-glucosaminidyl radical.

**6.** Use of a 1-arylsemicarbazide according to claim 3, characterised in that G signifies $PO_3MM'$, $SO_3M$, a carboxy-attached alkanecarboxylic acid, amino acid or oligopeptide radical and M and M' hydrogen, an alkali metal, alkaline earth metal or ammonium ion.

**7.** Process for the stabilisation of an enzyme substrate, from which, by enzymatic hydrolysis, a leuko compound can be formed which, in turn, can be reacted by oxidation to give a coloured material, characterised in that the substrate is brought into contact with a 1-arylsemicarbazide of the general formula I

Ar-NH-NH-$CONH_2$ (I)

in which Ar signifies an aryl radical possibly substituted by alkyl, alkoxy or halogen.

**8.** Diagnostic agent for the colorimetric determination of an enzyme, containing an enzyme substrate from which, by enzymatic hydrolysis, a leuko compound can be formed which, in turn, can be reacted by an oxidation agent to give a coloured material, characterised in that, for the stabilisation of the enzyme substrate, it contains a 1-arylsemicarbazide of the general formula I

Ar-NH-NH-$CONH_2$ (I)

in which Ar signifies an aryl radical possibly substituted by alkyl, alkoxy or halogen.

**9.** Diagnostic agent according to claim 8, characterised in that it additionally contains an oxidation agent.

**10.** Diagnostic agent according to one of claims 8 or 9, characterised in that it contains the components in carrier-bound form.

**Revendications**

**1.** Utilisation d'un 1-arylsemicarbazide de formule générale I

Ar-NH-NH-$CONH_2$ (I)

dans laquelle
Ar représente un reste aryle éventuellement substitué par un groupe alkyle, alcoxy ou halogéno,
pour la stabilisation d'un substrat d'enzyme à partir duquel on peut former, par hydrolyse enzymatique, un colorant leuco que l'on peut faire réagir à son tour avec un agent oxydant pour obtenir un colorant.

2. Utilisation d'un 1-arylsemicarbazide selon la revendication 1, en présence d'un agent oxydant.

3. Utilisation d'un 1-arylsemicarbazide selon l'une des revendications 1 ou 2, caractérisée en ce que le substrat d'enzyme est un dérivé d'aminophénol N- et O-substitué, de formule générale II

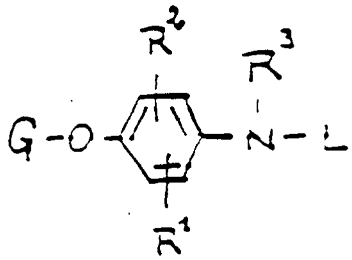

dans laquelle

G est un reste d'acide organique ou inorganique ou un reste glycoside,

$R^1$ et $R^2$, qui sont identiques ou différents, représentent un atome d'hydrogène, un atome d'halogène, $SO_3H$, $PO_3H_2$, ou un sel de ces groupes acides, un groupe hydroxy, nitro, carboxy, carboxamido ou cyano ou un groupe alkyle, alcényle, alcoxy, alkylsulfinyle, alkylsulfonyle, alcoxycarbonyle alkylcarbonyle, aryle ou aralkyle éventuellement substitué par un ou plusieurs restes hydroxy, carboxy, halogéno cyano, $SO_3H$ ou $PO_3H_2$ ou un sel de ces groupes acides, ou

lorsque les deux restes se trouvent sur des atomes de carbone voisins, ils représentent ensemble un group 1,4-butadiènediyle, qui est substitué une ou plusieurs fois par $SO_3H$, $PO_3H_2$ ou un sel de ces groupes acides, un groupe alkyle et/ou un groupe carboxy,

$R^3$ représente un atome d'hydrogène, CO-COOH, $SO_3H$, $PO_3H_2$ ou un sel de ces groupes acides, un groupe alkylcarbonyle substitué une ou plusieurs fois par un groupe halogéno, $CO_2H$, $SO_3H$ et/ou $PO_3H_2$ ou un sel de ces groupes acides, ou un groupe arylcarbonyle substitué une ou plusieurs fois par $SO_3H$, $PO_3H_2$ ou un sel de ces groupes acides, et

L représente un reste de formule générale III

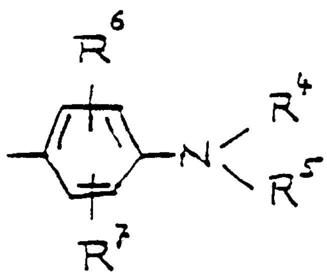

dans laquelle

$R^4$ et $R^5$, qui peuvent être identiques ou différents, représentent un groupe alkyle ou forment ensemble une chaîne hydrocarbonée saturée ayant 3-6 maillons, qui peut être interrompue par un atome d'oxygène, de soufre ou d'azote, le groupe alkyle ou la chaîne hydrocarbonée étant éventuellement substitué une ou plusieurs fois par un groupe hydroxy carboxy, alcoxycarbonyle, alcoxy, $SO_3H$ ou $PO_3H_2$, un sel de ces groupes acides ou un groupe halogéno, et

$R^6$ et $R^7$, qui peuvent être identiques ou différents, représentent un atome d'hydrogène, d'halogène, un groupe hydroxy ou carboxamido, ou un groupe alkyle, alcoxy, alkylcarbonyle, alcoxycarbonyle, aryle ou aralkyle éventuellement substitué par un ou plusieurs restes hydroxy, carboxy, halogéno, $SO_3H$ ou $PO_3H_2$ ou un sel de ces groupes acides, ou

L représente un reste d'un hétérocycle de type pyrazolo, de formule générale IV

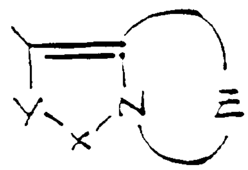

dans laquelle

X-Y représente $NR^8$-CO ou N = $CR^9$,

où

$R^8$ représente un atome d'hydrogène ou un groupe alkyle et

$R^9$ représente un groupe alkyle, alcényle, alcoxy, alkylthio, aryle, aralkyle, chacun éventuellement substitué par un groupe hydroxy, dialkylphosphinyle, carboxy, $SO_3H$, $PO_3H_2$, un sel de ces groupes acides et/ou un groupe alcoxycarbonyle ;
un groupe amino, qui est éventuellement substitué par un ou deux restes alkyle portant eventuellement un ou plusieurs groupe hydroxy, carboxy et/ou alcoxycarbonyle, ces restes pouvant également, dans le cas où le groupe amino est substitué par 2 restes alkyle, former ensemble un cycle qui peut être interrompu, en plus de l'atome N du groupe amino, éventuellement également par un atome d'oxygène, de soufre ou un autre atome d'azote, ou le groupe amino peut être substitué éventuellement par un ou deux groupes acyle, alcoxy et/ou aralcoxycarbonyle, $H_2N$-CO, alkyle, aralkyle et/ou arylcarbamoyle ; ou
un atome d'hydrogène, un groupe carboxy, alcoxycarbonyle, carboxamido ou halogéno, et

Z représente $NR^{10}$-N = N
où $R^{10}$ représente un atome d'hydrogène, un groupe alkyle ou aralkyle,

ou

Z représente une chaîne insaturée ayant 3 à 5 maillons, constituée d'atomes d'azote ou d'atomes de carbone, et éventuellement un ou plusieurs atomes d'azote ou de soufre, les atomes de carbone étant éventuellement substitués par un groupe alkyle, alcoxy, hydroxyalkyle, alkylthio, hydroxy, aralkyle, aryle, carboxy, carboxamido, alcoxycarbonyle, cyano, amino, qui peut être éventuellement substitué par un ou deux restes alkyle portant éventuellement un ou plusieurs restes hydroxy, carboxy et/ou alcoxycarbonyle, et/ou un groupe halogéno ainsi que des atomes d'azote, qui ne sont pas liés par une liaison double, qui sont éventuellement substitués par un groupe alkyle ou aralkyle, ou deux substituants voisins sur la chaîne forment ensemble éventuellement un groupe alkylène, qui à son tour, est éventuellement substitué par un groupe aryle, ou condensé, ou un reste tautomère correspondant.

**4.** Utilisation d'un 1-arylsemicarbazide selon la revendication 3, caractérisée en ce que G représente un reste de galactoside, de glucoside, de mannoside, de N-acétyl-glucosaminide ou un reste d'oligosaccharide ayant 2-10 motifs monosaccharide.

**5.** Utilisation d'un 1-arylsemicarbazide selon la revendication 3, caractérisée en ce que G représente un reste N-acétyl-$\beta$-D-glucosaminidyle.

**6.** Utilisation d'un 1-arylsemicarbazide selon la revendication 3, caractérisée en ce que

G représente $PO_3MM'$, $SO_3M$, un reste d'acide alcanecarboxylique, d'aminoacide ou d'oligopeptide lié avec la groupe carboxy, et

M et M' représentent un atome d'hydrogène, un ion alcalin, alcalinoterreux ou ammonium.

**7.** Procédé de stabilisation d'un substrat d'enzyme à partir duquel on peut former, par hydrolyse enzymatique, un colorant leuco, que l'on peut faire réagir à son tour avec un agent oxydant pour produire un colorant, caractérisé en ce que l'on met en contact le substrat avec un 1-arylsemicarbazide de formule générale I

$Ar\text{-}NH\text{-}NH\text{-}CONH_2$ (I)

dans laquelle
Ar représente un reste aryle éventuellement substitué par un groupe alkyle, alcoxy ou halogéno.

**8.** Agent de diagnostic pour la détermination colorimétrique d'une enzyme, contenant un substrat d'enzyme à partir duquel on peut former, par hydrolyse enzymatique, un colorant leuco, que l'on peut faire réagir à son tour avec un agent oxydant pour produire un colorant, caractérisé en ce que, pour stabiliser le substrat d'enzyme, il contient un 1-arylsemicarbazide de formule générale I

$Ar\text{-}NH\text{-}NH\text{-}CONH_2$ (I)

dans laquelle
Ar représente un reste aryle éventuellement substitué par un groupe alkyle, alcoxy ou halogéno.

**9.** Agent de diagnostic selon la revendication 8, caractérisé en ce qu'il contient en outre un agent oxydant.

**10.** Agent de diagnostic selon l'une des revendications 8 ou 9, caractérisé en ce qu'il contient les composants sous forme liée à un support.

Fig. 1

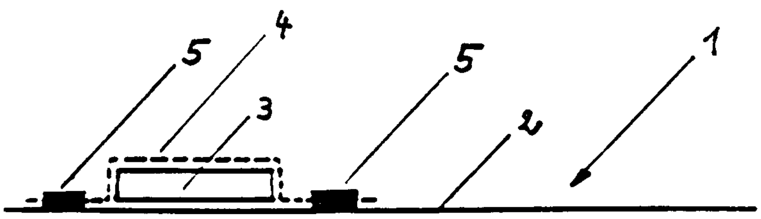


Fig. 2